# EUROPEAN PATENT APPLICATION

(11) **EP 2 047 842 A1**
(43) Date of publication of application: **15.04.2009**
(21) Application number: 07767584.1
(22) Date of filing: 26.06.2007
(51) Int. Cl.: A61K 8/73, A61K 8/06, A61K 8/36, A61K 8/42, A61K 8/92, A61Q 17/04, A61Q 19/00

(54) **COSMETIC AND METHOD FOR PRODUCTION THEREOF**

(30) Priority: 28.06.2006 JP 2006177500
(71) Applicant: Hakuto Co., Ltd, Shinjuku-ku, Tokyo 160-8910 (JP); Kanagawa University, Yokohama-shi, Kanagawa 221-8686 (JP)
(72) Inventor: NOHATA, Yasuhiro, Yokkaichi-shi, Mie 510-0007 (JP); TAJIMA, Kazuo, Yokohama-shi, Kanagawa 233-0001 (JP); IMAI, Yoko, Tokyo 158-0091 (JP); HORIUCHI, Teruo, Saitama 350-1308 (JP)
(74) Representative: Pautex Schneider, Nicole Véronique
(86) International application number: PCT/JP2007/062778
(87) International publication number: WO 2008/001757

(57) **Abstract**

A cosmetic exhibiting improved thermal stability and storage stability, which is prepared by using a three-phase emulsification-dispersing method in an aqueous phase. In the cosmetic containing an oily base material and/or a pigment as a component to be emulsification-dispersed, an emulsification-dispersing agent and water, the thermal stability and storage stability are improved, and the emulsification-dispersing agent composed of a polysaccharide having a particulate structure as a main component, and two or more kinds of components to be emulsification-dispersed are contained. The components to be emulsification-dispersed exhibit a dielectric constant ranging from 1 to 5 (F/m), and an inorganicity/organicity ratio ranging from 0 to 0.5.

## Description

### TECHNICAL FIELD

The present invention relates to a cosmetic containing a component to be emulsification-dispersed, which is composed of two or more kinds of oily base materials, or composed of two or more kinds of oily base materials and a pigment, an emulsification-dispersing agent composed of a polysaccharide, and water, and exhibiting thermal stability and storage stability.

### BACKGROUND ART

The cosmetics are required to exhibit many and various effects according to the kind thereof, so that various many components are contained therein. For example, an oily base material or a pigment, which has the cleaning effect, water-retaining effect, scrubbing effect, emollient effect and protection effect against skin, and performs the functions of improving the spreading ability, smooth feeling, glossiness, adhering properties, etc. of the cosmetic, and addition components such as an organic solvent, a moisturizer, an astringent, a bleaching agent, a UV preventive agent, an antioxidant agent, a perfume, etc. are contained.

Where this oily base material or the pigment is used in the cosmetic, conventionally, emulsification and dispersion thereof has been performed by selecting and using various kinds of surfactants according to a required HLB value of the oily base material and the properties of a surface of the pigment. And different kinds of surfactants as the emulsification-dispersing agent must be selected according to required HLB values thereof in the case of oil-in water (O/W) emulsions being produced and the case of water-in-oil (W/O) emulsions being produced, and in order to obtain the sufficient thermal stability and the sufficient storage stability, the most suitable surfactant must be selected out of many and various kinds of surfactants (see Non patent documents 1 through 4, for example).

The cosmetics are required to perform many functions so that various oily base materials, pigments and addition components are required to be stably maintained in one cosmetic, but it is very difficult to satisfy this requirement. For example, silicone oil is chemically stable and safe, and odorless with little stickiness, and it has a low surfacial tension with good spreading ability, and has smooth feel so as to have been blended in many cosmetics such as creams, milky lotions, lotions, gels, etc.

Where dimethyl polysiloxan is blended in the cosmetic containing stearic acid (organic acid) as the oily base material, cetanol (higher alcohol), and titanium dioxide and iron oxide (inorganic particles) as the pigment, dimethyl polysiloxan that is strong in hydrophobic properties, and inferior in compatibility with a general-purpose hydrocarbon-base surfactant so that it has been difficult to be stably emulsified in an aqueous solution. And there may be the case where it has become further difficult to emulsify stearic acid (fatty acid) and cetanol (higher alcohol), and emulsifiication-disperse inorganic particles such as titanium dioxide and iron oxide.

Therefore, in order to select an emulsifying agent most suitable to the cosmetic, very troublesome and great labors have been required, but in the cosmetic in which various kinds of oily base materials are intermixed, in many cases, it has been difficult to obtain a stable emulsion.
The conventional emulsifying method using surfactants is intended to adsorb surfactants on surfaces between oil and water, thereby decreasing the surface energy thereof, and consequently, in order to decrease the surfacial tension therein, a large amount of emulsifiers exhibiting high surface activity has been needed. Under these circumstances, the skin irritation of the surfactants used in the cosmetic has been pointed out, and the reduction thereof has been demanded. In addition, the surfactants having high emulsifying abilities are generally low in biodegradation so that, recently, it has been pointed out the problem of the environmental contamination such as foaming of waste water due to the surfactants, and consequently, reduction of the use thereof has become a social demand.

Under the above circumstances, various improved methods such as the emulsifying method using a reduced amount of surfactants, and the emulsifying method using no surfactant, etc. have been proposed. For example, there is disclosed the method of emulsifying and dispersing the oily base material (or the pigment) into an oil-in water (O/W) emulsion by the three-phase emulsification-dispersing method using 6 to 15 moles of ethylene oxide adducts of caster oil with a specific structure, and a cationic surfactant (Publication of unexamined Japanese patent application No. 2004-130300). In order to obtain the emulsification-dispersing properties equivalent to those obtained with the conventional emulsification-dispersing method using the surfactants, the total amount of 6 to 15 moles of ethylene oxide adducts of caster oil and the cationic surfactant may be made smaller than the amount of the conventional surfactants, whereby the amount of the surfactants is reduced. With this method, ethylene oxide adduct of caster oil, which is insoluble in water, is considered to achieve the function as the emulsifier.

As a result of the examination of various materials exhibiting similar functions to the ethylene oxide adducts of caster oil, the present inventors have invented the cosmetic exhibiting high emulsification-dispersing stability, which is produced by the three-phase emulsification-dispersing method using a specific polysaccharide as an emulsification-dispersing agent without using any surfactant (see Non patent documents 5 and 6, for example). This method does not require troublesome works such as the selection of many kinds of surfactants according to the required HLB value of the oily base material and the properties of a surface of the pigment so as to be a convenient and excellent method. Where the component to be emulsified is composed of a single substance, the three-phase emulsification-dispersing method using a specific polysaccharide provides a stable emulsion, but where two or more kinds of oily components are blended, the formation of emulsions excellent in thermal stability and storage stability is insufficient, and the improvement thereof has been strongly demanded. In particular, where a specific pigment is blended together, the emulsion thereof may be damaged in several minutes, and consequently, it has been difficult to apply this method to the preparation of cosmetics in which various kinds of components are blended.

Patent document 1: Publication of unexamined Japanese patent application No. 2004-130300
Non patent document 1 :"Emulsion Science" Edited by P. Sherman, Academic Press Inc. (1969)
Non patent document 2:"Microemulsions-Theory and Practice" Edited by Leon M. price, Academic Press Inc. (1977)
Non patent document 3:"Technique of Emulsification and Solubilization" edited by Susumu Tsuji, Kougakutosho Ltd. (1976)
Non patent document 4:"Developing Technique of Functional Surfactant" published by CMC Publishing Co., Ltd. (1998)
Non patent document 5: Abstract of The 16 year Divisional Meeting on Colloid and Surface Chemistry, the Chemical Society of Japan 2C04 "Novel Emulsification with Soft Nanoparticles: the Principle of Three-Phase Emulsification"
Non patent document 6: Abstract of The 16 year Meeting of the Japan Oil Chemical Society of Japan P102 "Emulsion and stability of hydrocarbon and silicone oil with the three-phase emulsification"

### DISCLOSURE OF THE INVENTION

### PROBLEMS TO BE SOLVED BY THE INVENTION

The present inventors have intensively studied the thermal stability and the long-term stability of the cosmetic containing two or more kinds of oily base materials, or two or more kinds of oily base materials and a pigment, as a component to be emulsification-dispersed, a polysaccharide as an emulsificatian-dispersing agent, and water, and, as a result, they have found that by using the oily base material having a specific dielectric constant, and a specific inorganicity/organicity ratio in the three-phase emulsification-dispersing method in an aqueous phase using a specific polysaccharide, the storage stability of the cosmetic containing two or more kinds of oily base materials is improved, and have completed the present invention.
Accordingly, the object of the present invention is to provide a cosmetic containing two or more kinds of oily base materials, or containing two or more kinds of oily base materials and a pigment, along with water, with improved thermal stability and improved storage stability, using the three-phase emulsion dispersing method.

### MEANS FOR SOLVING THE PROBLEMS

The present invention according to claim 1 is the cosmetic containing an emulsification-dispersing agent composed of a polysaccharide having a particulate structure as a main component, and a component to be emulsification-dispersed, wherein the component to be emulsification-dispersed is composed of two or more kinds of components to be emulsification-dispersed, each having a dielectric constant ranging from 1 (F/m) to 5 (F/m), and an inorganicity/organicity ratio ranging from 0 to 0.5.

In accordance with the present invention of claim 1, in the cosmetic containing an emulsification-dispersing agent composed of a polysaccharide having a particulate structure as a main component, and components to be emulsification-dispersed, the component to be emulsification-dispersed has a dielectric constant ranging from 1 (F/m) to 5 (F/m), and an inorganicity/organicity ratio ranging from 0 to 0.5. Therefore, the cosmetic using the polysaccharide having a particulate structure and the component to be emulsification-dispersed, which has a dielectric constant and a inorganicity/organicity ratio within the above-described ranges, can form an emulsified and dispersed system excellent in thermal stability and long-term stability against surfaces between an oily base material mixture as the component to be emulsification-dispersed, and water. Consequently, the cosmetic that is stable in emulsification and dispersion over a long period of time and in a wide temperature range can be obtained. In addition, by using the emulsification-dispersing agent composed of the polysaccharide having a particulate structure as a main component, and the components to be emulsification-dispersed (oily base material) of the present invention, a stable emulsified state can be maintained even where ceramide powder, zinc oxide powder, titanium dioxide powder, mica powder as powders having surface activity, or such components as to damage the emulsification stability, such as magnesium salt ascorbic acid phosphate, ascorbic acid glucoside, etc., which are easy to deposit, are contained in the component to be emulsification-dispersed.

The present invention according to claim 2 is the cosmetic wherein the emulsification-dispersing agent composed of the polysaccharide having the particulate structure as the main component adheres to a periphery of the component to be emulsification-dispersed into a layer, thereby defining an intermediate layer.

In accordance with the present invention of claim 2, the polysaccharide having a particulate structure adheres to the periphery of the component to be emulsification-dispersed into a layer with van der Waals' force to form the intermediate layer irrespective of the properties of the components to be emulsification-dispersed so that the components to be emulsification-dispersed can be stably emulsified, and the emulsion state can be maintained. Namely, emulsification-dispersing agent phases are formed in surfaces between the components to be emulsification-dispersed and solvents so that the components to be emulsification-dispersed are difficult to coalesce with each other after emulsification, and accordingly, resultant emulsification-dispersed layers are very excellent in thermal stability and storage stability irrespective of the kinds of the components to be emulsification-dispersed.

The present invention according to claim 3 is a cosmetic in which the polysaccharide with a particulate structure has an average particle diameter ranging from 8 nm to 500 nm.

In accordance with the present invention of claim 3, there can be obtained an emulsification-dispersed state in which the components to be emulsification-dispersed are stable with the average particle diameter ranging from 8 nm to 500nm. When the average particle diameter is 8 nm or less, the adhering function due to the van der Waals' force is decreased, and consequently, monoparticles in an emulsification-dispersed state become difficult to adhere to surfaces of oil drops, whereas when the particle diameter is greater than 500 nm, a stable emulsion cannot be maintained.

The present invention according to claim 4 is a cosmetic wherein the polysaccharide with a particulate structure is a polysaccharide formed into a monoparticle.

in accordance with the present invention of claim 4, since the polysaccharide is formed into a monoparticle, a large number of monoparticles adhere to a periphery of the component to be emulsification-dispersed into a layer to form an independent phase, and consequently, the emulsification-dispersing agent composed of monoparticles stably exists as a third phase so that the emulsion state can be maintained over a long period of time.

The present invention according to claim 5 is a cosmetic wherein the polysaccharide with a particulate structure contains at least one of fucose, glucose, glucuronic acid and rhamnose as a constituent monosaccharide, and further contains fucose and/or rhamnose in a side chain thereof.

In accordance with the present invention of claim 5, since these polysaccharides are contained as the constituent monosaccharide, and fucose and/or rhamnose are contained in a side chain thereof, the polysaccharide with a particulate structure retains the components to be emulsification-dispersed inside thereof to form a construction of which an outer surface forms a phase exhibiting hydrophilic properties and hydrophobic properties, thereby forming a dispersion structure composed of components to be emulsification-dispersed-polysaccharide with a particulate structure-solvents.

The present invention according to claim 6 is a cosmetic wherein the polysaccharide with a particulate structure includes at least the polysaccharide represented by the following formula (1).

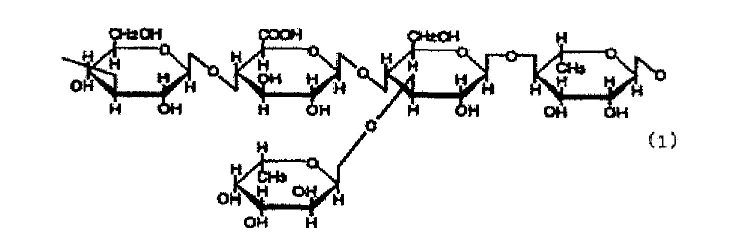

In accordance with the present invention of claim 6, since the polysaccharide represented by the formula (1) is contained, the particulate structure can be formed, and since the outer surface thereof contains hydrophilic groups such as OH groups, etc., the outer surface exhibits hydrophilic properties and hydrophobic properties due to the adhesion of the polysaccharide with the particulate structure to the components to be emulsification-dispersed, whereby a dispersion structure composed of components to be emulsification-dispersed- polysaccharide with a particulate structure-solvents can be formed.

The present invention according to claim 7 is a cosmetic wherein the emulsification-dispersing agent containing the polysaccharide with a particulate structure as a main component exists in the weight ratio of from 1 : 50 to 1 : 1000 to the components to be emulsification-dispersed.

In accordance with the present invention of claim 7, by virtue of the particulate structure, even when a small amount of emulsification-dispersing agent is used against the components to be emulsification-dispersed, a stable emulsification-dispersed substance can be obtained. Where the weight ratio of the emulsification-dispersing agent to the components to be emulsification-dispersed is 1 : 50 or less, the function of the emulsification-dispersing agent as the emulsion particles disappears to increase the viscosity of the system, and where the weight ratio is 1 : 10000 or more, it becomes difficult to form such a particulate structure. For these reasons, the preferable weight ratio ranges from 50 to 1000.

The present invention according to claim 8 is a cosmetic wherein the content of the polysaccharide with a particulate structure ranges from 0.001% by weight to 1 % by weight relative to the total amount of the cosmetic.

According to the present invention of claim 8, the content of the polysaccharide with a particulate structure ranges from 0.001 % by weight to 1% by weight relative to the total amount of the cosmetic so that the components to be emulsification-dispersed can be dispersed stably.

The present invention according to claim 9 is a cosmetic wherein the components to be emulsification-dispersed include two or more kinds of a lake pigment, organic pigment, color pigment, white pigment, inorganic pigment such as extender pigment, nacreous pigment, metallic luster pigment, silica frit pigment, metal coated inorganic pigment, resin pigment, high polymer powders, and functional pigment.

According to the present invention of claim 9, since the above-described emulsion-dispersing agent is used, two or more kinds of the above-described components to be emulsion-dispersed can be dispersed stably, even when the above-described pigments are contained as the components to be emulsification-dispersed.

The present invention according to claim 10 is a cosmetic that does not contain any one of cationic surfactants, anionic surfactants and nonionic surfactants composed of alkylene (carbon atoms: 2 to 4) oxide adduct.

According to the present invention of claim 10, since the cosmetic does not contain any one of cationic surfactants, anionic surfactants and nonionic surfactants composed of alkylene (carbon atoms: 2 to 4) oxide adduct, the blending properties into skin are improved, satisfactory feeling can be obtained upon using, and not only the influence to skin contacting the cosmetic but also the influence to natural environment can be reduced over a long period of time.

The present invention according to claim 11 is a cosmetic wherein the polysaccharide is granulated into randomly scattering particle diameters.

According to the present invention of claim 11, since the polysaccharide is granulated into randomly scattering particle diameters, the polysaccharide does not have any crystal structure so that the cosmetic in which the emulsion state of the components to be emulsification-dispersed is stable can be obtained.

The present invention according to claim 12 is a cosmetic containing urea in an amount ranging from 0.1 % by weight to 10 % by weight relative to the total amount of the cosmetic.

According to the present invention of claim 12, since the cosmetic contains urea in an amount ranging from 0.1 % by weight to 10 % by weight relative to the total amount of the cosmetic, the formation of the particulate structure of the components to be emulsification-dispersed is accelerated.

### EFFECTS OF THE INVENTION

The cosmetic in accordance with the present invention, which uses a polysaccharide having a particulate structure and a specific oily base material as a component to be emulsification- dispersed forms a emulsified and dispersed system excellent in thermal stability and storage stability against surfaces between a mixture of the oily base material and water so that the cosmetic that is stable in emulsification and dispersion over a long period of time and in a wide temperature range can be obtained. In addition, where the component to be emulsification-dispersed (oily base material) of the present invention and an emulsification-dispersing agent containing the polysaccharide having the particulate structure as a main component are used, a stable emulsion state can be maintained even if the component to be emulsification-dispersed, especially, ceramide powder, zinc oxide powder, titanium dioxide powder, mica powder as the powder exhibiting surface activity, or a component damaging the emulsion stability, such as magnesium salt ascorbic acid phosphate, ascorbic acid glucoside, etc. as an additive are contained. In addition, since the surfactant is not used as the emulsification-dispersing agent, the blending properties into skin is also improved, satisfactory feeling upon using can be obtained, and not only the influence to skin contacting the cosmetic but also the influence to natural environment can be reduced over a long period of time.

### BRIEF EXPLANTIION OF THE DRAWINGS

FIG. 1 is a diagram explaining an emulsification mechanism, FIG. 1(A) is a diagram explaining an adsorption mechanism of a monomolecular film of a conventional surfactant, and FIG. 1(B) is a diagram explaining an adhesion mechanism of a polysaccharide with a particulate structure.
FIG. 2 is a diagram explaining a phenomenon caused by thermal convection of emulsified and dispersed particles, FIG. 2(A) is a diagram explaining a phenomenon caused by thermal convection in a conventional adsorption molecule type, and FIG. 2(B) is a diagram explaining a phenomenon caused by thermal convection in an emulsifying agent phase adhesion type.
FIG. 3 is a photograph taken by an electron microscope, which explains an emulsified and dispersed state of a component to be emulsification-dispersed of the present invention.

### BEST MODE FOR CARRYING OUT THE INVENTION

The present invention is a cosmetic containing two or more kinds of oily base materials, or containing two or more kinds of oily base materials and a pigment, as a component to be emulsification-dispersed, along with water, and such a cosmetic is obtained by emulsification-dispersing the component to be emulsification-dispersed, which is composed of the two or more kinds of oily base materials, or the two or more kinds of oily base materials and the pigment with a three-phase emulsion dispersion method using the oily base material with a specific dielectric constant, and a specific inorganicity/organicity ratio, and an emulsification-dispersing agent containing a polysaccharide with a particulate structure as a main component, and exhibits improved thermal stability and storage stability.

The cosmetic in accordance with the present invention contains a component to be emulsification-dispersed, which is composed of two or more kinds of oily base materials, or two or more kinds of oily base materials and a pigment, along with water, and such a cosmetic is obtained by emulsification-dispersing the component to be emulsification-dispersed in water with an emulsification-dispersing agent containing a polysaccharide with a particulate structure as a main component. More specifically, the cosmetic has various configurations such as a milky lotion, cream, paste, gel, etc. Examples thereof include base cosmetics such as a face cleaning cream, cleansing cream (foam), lotion, pack, massage cream, moisturizing milky lotion, moisturizing cream, and lip cream; finish cosmetics such as foundation cream, eye shadow, mascara and lip rouge; hair cosmetics such as hair gel, hair cream, shampoo, rinse, hair treatment, hair conditioner, hair mousse, hair liquid and set lotion; fragrance cosmetics such as perfume (perfume and parfum), eau de parfum (perfume cologne), eau de toilette (perfume de toilette, parfum de toilette) and eau de cologne (cologne, fresh cologne); and sunburn preventive cosmetics such as sunscreen gel, sunscreen cream and sunscreen milky lotion.

The oily base material as the component to be emulsification-dispersed is generally an organic substance adapted to be blended in a cosmetic in order to achieve protecting effect, moisturizing effect, moisture retaining effect, nourishing effect , etc. against skin, and the oily base material as an emulsification-dispersing agent in accordance with the present invention has a dielectric constant ranging from 1 (F/m) to 5 (F/m), and a ratio of the inorganicity to the organicity (hereinafter will be refer to "inorganicity/organicity ratio") ranging from 0 to 0.5. The inorganicity and the organicity of the oily base material in accordance with the present invention correspond to the inorganicity/organicity ratio that is proposed in Organic conception diagram (Reference document: "Systematic organic qualitative analysis" Fujita, Kazamashobo Co., Ltd. (1974), "Organic conception diagram- basis and application" Yoshio Koda, SANKYO PUBLISHING Do., Ltd.(1984)), and are obtained by calculating the oraganicity and inorganicity based on a prescribed numerical value with respect to the composition elements of organic compounds and the functional groups, and obtaining the ratio thereof. Where two or more kinds of oily base materials are used, the dielectric constant of respective oily base materials and the inorganicity/organicity ratio are required to be in the range of the present invention. The above-described ranges of the dielectric constant and the inorganicity/organicity ratio are ranges found from actual results, and where the dielectric constant does not range from 1 (F/m) to 5 (F/m), and the inorganicity/organicity ratio does not range from 0 to 0.5, the effects of the present invention cannot be obtained.

Examples of the oily base material include hydrocarbon having carbon atoms of 6 through 20, such as hexane, cyclohexane, octane, decane, dodecane, icosane, etc.; wax such as petroleum waxe, etc. halogenated hydrocarbon such as 1-bromo octane, fluorocarbon, etc; higher alcohol having carbon atoms of 14 through 22, such as tetradecanol, hexadecanol, octadecanol, behenyl alcohol, etc.; higher fatty acid having carbon atoms of 14 through 22 such as myristic acid, palmitic acid, stearic acid, oleic acid, behenic acid, etc.; uryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol, 2-decyl-tetra-decinol, cholesterol, phytosterol, sitosterol, lanosterol, POE cholesterol ether, mono stearyl glycerin ether (batyl alcohol), etc., higher fatty acid ester having carbon atoms 14 through 22 such as ethyl myristate, ethyl stearate, methyl oleate, methyl behenylate, etc.; oil such as caster oil, rapeseed oil, sesame oil, etc.; fragrance such as turpentine oil, rose oil, camellia oil, etc., dimethyl siloxane, methyl phenyl siloxane, and dimethyl polyoxysilane-polyoxyalkylene copolymer, etc., and two or more of these materials may be used in combination.

The pigment as the component to be emulsification-dispersed is a substance adapted to be blended in the cosmetic for achieving filming effect (covering effect), color effect, etc., and examples thereof include inorganic mineral-base powder such as titanium oxide, iron oxide (inorganic particles), zinc chloride, etc.; lake pigment, organic pigment, color pigment, white pigment, extender pigment, nacreous pigment, metallic luster pigment, glass flake pigment, metal coated inorganic pigment, resin pigment, high polymer powder (pigment), functional pigment, etc. One or more of these pigments are used.

There are two kinds of lake pigment. One is the pigment obtained by making a water-soluble dye into salts of calcium, etc. that is insoluble in water. Examples thereof include red No.202, red No.204, red No.206, red No.207, red No.208, red No.220, etc. The other one is the pigment that is made water-insoluble with aluminum sulfate, zirconium sulfate, etc., and is adsorbed on alumina. Examples thereof include yellow No. 5, red No. 230, etc.
The organic pigment is composed of a colored powder that has no hydrophilic group in its molecular structure, is not dissolved in water, oil and solvents, and is excellent in coloring force and light resistance. Examples thereof include red No.228 of azo pigment, red No.226 of indigo pigment, blue No.404 of phthalocyanine pigment, etc.

Examples of the inorganic pigment include iron oxides having different colors, such as red iron oxide, yellow iron oxide, black iron oxide, etc., ultramarine blue, milori blue, chromium oxide, chromium hydroxide, magnesium oxide, cobalt oxide, cobalt titanium oxide carbon black, manganese violet, cobalt violet, etc.
White pigment is used for coloring, covering or the like, and examples thereof include titanium dioxide and zinc oxide. Titanium oxide· sintered titanium oxide, zinc oxide· sintered zinc oxide may be subjected to the surface treatment with normally well known methods such as the silica treatment, the treatment with silicone compounds such as dimethyl polysiloxane, methylhydrogen-polysiloxane, trimethyl siloxysilicate, etc., the treatment with furuolo compounds such as perfluoro polyether phosphate, perfluoro alkyl phosphate, fluorine modified silicon, etc., the treatment with metallic soap such as zinc laurate, etc., the treatment with amino acids such as N-long chain acyl amino acid, etc., the treatment with oils such as higher fatty acids, higher alcohols, esters, waxes, etc.

Extender pigment is used not for coloring but for maintaining configurations of products, controlling extensibility, adhesion and luster thereof, and adjusting color tone thereof, and examples thereof include mica-based pigment such as mica, muscovite, synthesis mica, phlogopite, red mica, biotite, lithia mica, etc., clay minerals such as sericite, talc, kaolin, montmorillonite, zeolite, etc., synthetic inorganic powders such as magnesium carbonate, calcium carbonate, silicic acid, anhydrous silicic acid, aluminum silicate, magnesium silicate, magnesium aluminum silicate, sulfur-containing aluminum silicate, calcium silicate, barium silicate, strontium silicate, aluminum oxide, barium sulfate, etc.

The nacreous pigment is the pigment adapted to be used for giving pearl-like luster, rainbow colors, and metallic feeling to products, and examples thereof include mica covered with titanium dioxide, fishes scale foil, bismuth oxychloride, etc. In addition, pigment covered with iron oxide in place of titanium oxide, pigment having a covering layer of titanium oxide, which is covered with another pigment of a transparent different color, etc. are used.
Examples of the metallic luster pigment include aluminum powder, brass powder, copper powder, tin powder, gold dust, silver powder, etc., and colored metallic powder pigment prepared by coloring these metallic powders.
The silica frit pigment is the pigment in which flake-shaped glass is covered with metal or a metallic oxide.
The metal-coated inorganic pigment is an inorganic pigment coated with metal and/or metal oxide by metal evaporation, etc., and examples thereof include aluminum coated with iron oxide, mica coated with iron oxide, micaceous iron oxide coated with aluminum-manganese, etc.

The resin pigment is composed of resin flakes prepared by coloring a resin film and cutting the colored resin film into flakes, examples of the resin pigment include polyester film powder, polyethylene terephthalate aluminum epoxy laminate film powder, polyethylene terephthalate polyolefine lamination film powder, polymethyl methacrylate, polyethylene terephthalate polymethyl methacrylate lamination powder, nylon powder, etc.
Examples of the functional pigment include boron nitride, synthetic boron gold phlogopite, photochromic pigment, composite fine-grain powder, etc.

The configuration of the brightening pigment in accordance with the present invention is not limited specifically, but may be arbitrarily selected from a grain shape, sheet shape, rod shape, etc. according to the object and kind of pigment to be used. The size of the pigment is not limited specifically, but may be arbitrarily selected according to the object and kind of pigment to be used. Normally, in the case of a particle shape, the pigment having a mean particle diameter ranging from 0.01 µ m to 5000µ m is used, and in the case of a flake shape or rod shape, the pigment having a longer diameter ranging from 0.1 µ m to 5000µ m is used.

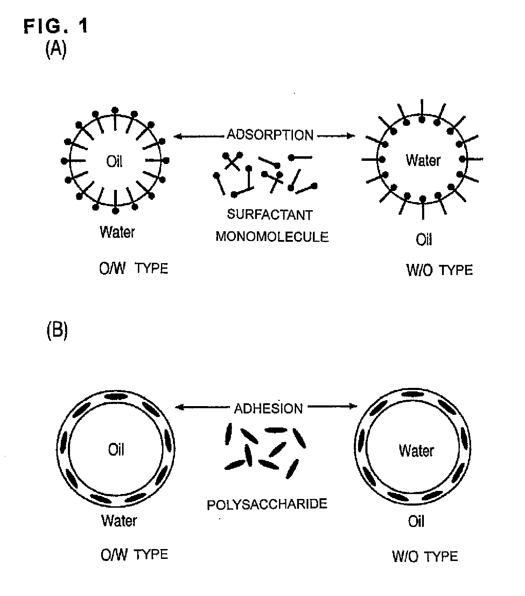

Hereinafter, the cosmetic of the present invention will be explained with reference to the cosmetic containing oily base materials, while comparing with the conventional cosmetic. FIG. 1 shows a schematic diagram of an emulsifying method using surfactants to be contained in the conventional cosmetic and a three-phase emulsifying method using emulsification-dispersing agent to be used in the cosmetic of the present invention.
In the conventional emulsifying method using surfactants, as shown in FIG. 1(A), the surfactant has hydrophilic groups and hydrophobic groups which respectively have properties different from each other in each monomolecule. The hydrophobic groups of the surfactant exhibit compatibility with oil phase, whereas the hydrophilic groups are arranged to be oriented outside the oil phase, thereby readily blending with water. Consequently, they are mixed in a water medium homogeneously to form an O/W type emulsion. In addition, the hydrophilic groups of the surfactant are oriented against water particles, whereas the hydrophobic groups of the surfactant are arranged to be oriented outwardly of the water particles, readily blend with oil, and are mixed in an oil medium homogeneously, thereby forming a W/O type emulsion.

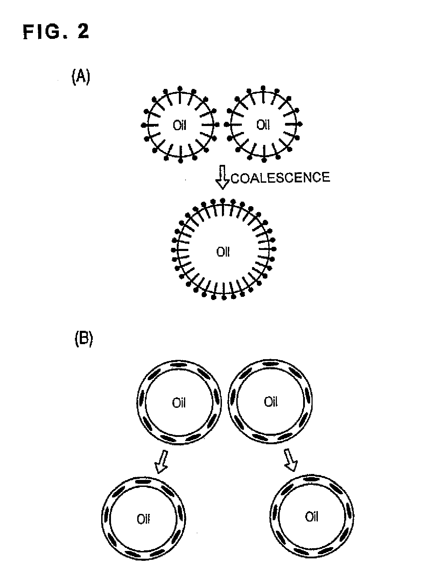

However, with the conventional emulsifying method, the surfactants are adsorbed on oil surfaces to form emulsion films like monomolecules films so that there occurs such a disadvantage that the physical properties of an surface vary according to the kind of the surfactant. In addition, as shown in FIG. 2(A), oil drops coalesce with each other due to thermal impact thereof, and consequently, the dimensions of the oil drops gradually increase and at last, there occurs the separation into oil and an aqueous solution of surfactants. In order to prevent such separation, the method of granulating the oil drop particles such as the method of forming microemulsions is needed, and in this case, there occurs the disadvantage that a large amount of surfactants must be used.

Since the hydrophobic components of the oily base material have hydrophobic properties and hydrophilic properties due to their chemical structures, and consequently, in order to emulsify the same, conventionally, the surfactant has been selected based on indexes of the solubility relative to the oily base material to be emulsified, such as the HLB value, etc., and the most suitable one has been selected. However, since the cosmetics contain many oily components, it is very difficult to select one surfactant or a combination of a plurality of surfactants, which are suited to all of the oily components, and consequently, in many cases, experiences must be needed. In addition, the amount of the surfactants to be used normally becomes as high as 5 through 20 %. This high content causes problems in thermal stability and physiological stability of the cosmetic.

Under the above circumstances, by using a new emulsifying method (three-phase emulsifying method) of making particles of an emulsification-dispersing agent containing a polysaccharide with a particulate structure as one component thereof adhere to an oil phase and water particles in the cosmetic, as shown in FIG. 1 (B), thereby forming a three phase structure composed of water phase-emulsification-dispersing agent phase-oil phase and forming an emulsion, the surface energy is prevented from decreasing due to the adsorption of the emulsifying agent against surfaces between oil and water, as is different from the conventional emulsification-dispersing method using surfactants, and consequently, as shown in FIG. 2(B), coalescence caused by thermal impact is difficult to occur, whereby the cosmetic in which the emulsion is stabilized over a long period of time can be obtained.

In this case, the reasons have not been clarified accurately, but when two or more kinds of the oily base materials are used, the effect of the present invention cannot be obtained, if the dielectric constant is outside the range from 1 (F/m) to 5 (F/m), and the inorganicity/organicity ratio is outside the range from 0 to 0.5. It can be assumed that when the oily base material outside these ranges is contained, the van der Waals' force is not sufficiently obtained between the polysaccharide with a particulate structure and the oily base material so that the polysaccharide with the particulate structure does not adhere to a surface of the oily base material stably, and consequently, the three phase structure composed of water phase-emulsification-dispersing agent phase-oil phase is damaged, whereby the emulsified state also disappears.
In addition, in the cosmetic containing the oily base material and water, by emulsification-dispersing the oily base material with the three phase emulsification-dispersing method in an aqueous phase using an emulsification-dispersing agent composed of a polysaccharide with a particulate structure as a main component, the cosmetic excellent in thermal stability and storage stability can be obtained.

The emulsification-dispersing agent adapted to effect the three-phase emulsification is an emulsification-dispersing agent containing the polysaccharide with a particulate structure as a main component (hereinafter will be referred to "emulsification-dispersing agent of the present invention"). The polysaccharide with a particulate structure is the polysaccharide hydrated and dissolved and/or the polysaccharide hydrated but undissolved, which is stably dispersed in an aqueous phase, that is the polysaccharide of which long chain molecules have a spherical configuration or a ball-like configuration, and are dispersed in an aqueous phase stably, but not the polysaccharide undissolved or deposited in an aqueous phase.

And the polysaccharide made into monoparticles is the polysaccharide in the state that a single spherical (or ball-like) polysaccharide exists, and in order to achieve the three-phase emulsification-dispersing effect due to the adhesion to the surface of the component to be emulsification-dispersed, it is preferable that the polysaccharide is composed of monoparticles. Where a plurality of polysaccharides, each having a particulate structure, are assembled, and consequently, an apparent single monoparticle of polysaccharide exists, the three-phase emulsification-dispersing effect decreases, which is less preferable. The preferred average particle diameter of polysaccharide with a particulate structure ranges from 8 nm to 500 nm. Where the particle diameter is less than 8 nm, the attracting action due to the van der Waals' force decreases so that the polysaccharide may become difficult to adhere to surfaces of the component to be emulsification-dispersed. And where the particle diameter is greater than 500 nm, a stable emulsion may not be maintained.

The polysaccharide to be used in the present invention, is the polysaccharide composed of at least fucose, glucose, glucuronic acid and rhamnose as a constituent monosaccharide, and containing fucose and/or rhamnose as a side chain. It is preferable that the polysaccharide is composed of fucose, glucose, glucuronic acid and rhamnose as constituting monosaccharides, and containing fucose and/or rhamnose as a side chain, and it is more preferable that, as shown in the following formula (1), the polysaccharide has a main chain with a repetition construction composed of glucose, glucuronic acid and rhamnose and one fucose diverges from one glucose in the main chain.

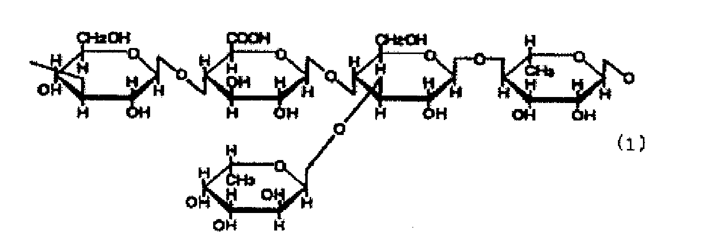

The polysaccharide represented by the formula (1) can be obtained as a product of a microorganism of Alcaligenes latus strain B-16 (FERM BP-2015), for example. The microorganism of Alcaligenes latus strain B-16 is cultured by a normal microbes culturing method, and, after cultured, organic solvents such as acetone, ethanol and isopropyl alcohol are added to a culture liquid. As a result, polysaccharide precipitates as an insoluble substance. The precipitation of polysaccharide is separated, thereby obtaining a polysaccharide.

Generally, microorganisms produce two or more kinds of polysaccharides. Other kinds of polysaccharide than the polysaccharide of the present invention may be included provided that they do not obstruct the effects of the present invention. For example, it has been proved that the polysaccharides produced by a microorganism Alcaligenes latus strain B-16 include at least two kinds of polysaccharides, and the molar ratio of the constituting monosaccharides of polysaccharides which are separated from culture liquid is:fucose : glucose : glucuronic acid : rhamnose = 1 : (0.5 to 4) : (0.5 to 2) : (0.5 to 2). When these two kinds of polysaccharides are separated from each other, one kind of polysaccharide is a polysaccharide having a construction wherein one fucose diverges from one glucose in the main chains with a repeated construction, which is composed of glucose, glucuronic acid and rhamnose, as shown in the formula (1), and another kind of polysaccharide is a polysaccharide of which repetition unit is composed of fucose and mannose. The former is the polysaccharide of the present invention, has a constituting ratio of fucose, glucose, glucuronic acid and rhamnose which is 1 : 2 :1 : 1, and is a high molecular component having a molecular weight of about 10⁹ (see the Japan Agricultural Chemistry Society, 1998th year Large Meeting, Summary, P. 371]. The latter is the polysaccharide having a repetition construction of fucose and mannose of 1 : 1, and is a low molecular component having a molecular weight of 10³ to 10⁷ (see Y. Nohata, J. Azuma, R. Kurane, Carbohydrate Research 293, (1996) 213 to 222). This low molecular component is not within the scope of the polysaccharide of the present invention, but does not obstruct the stabilizing effect of the present invention so that it may be contained in the cosmetic.

This polysaccharide has been sold on market as Alcasealan [trade name, INCl same: Alcaligenes Polysacchaides, manufactured by HAKUTO CO., LTD.]. In addition, by using sphingomonas trueperiSPH-011 (FERM BP-08582) or SPH-012(FERM BP-08579) in place of microorganism Alcaligenes latus strain B-16 (FERM BP-2015), the polysaccharide of the present invention can be obtained.

The content of the emulsification-dispersing agent of the present invention ranges from 1:50 to 1:1000, and preferably from 1:100 to 1:500, in the weight ratio to the component to be emulsification-dispersed, in the case of the polysaccharide with a particulate structure, which is contained in the emulsification-dispersing agent. When the weight ratio of the emulsification-dispersing agent to the component to be emulsification-dispersed is 1:50 or less, the content of the emulsification-dispersing agent increases to raise costs, and in the case of 1:1000 or more, the particulate structure becomes difficult to be formed so that the preferable weight ratio ranges from 50 to 1000. Where various many components are blended, like the cosmetics, it is further efficient to perform the emulsification and dispersion by two steps.

And the content of the polysaccharide of the present invention in the cosmetic may be arbitrarily determined according to the kind of an objective cosmetic, and required emulsification-dispersion stability for the cosmetic, and normally ranges from 0.001 % by weight to 1 % by weight, preferably from 0.01 % by weight to 0.5 % by weight, and more preferably from 0.05 % by weight to 0.2 % by weight relative to the total amount of the cosmetic. When the concentration of the polysaccharide is less than 0.001 % by weight, there may be the case sufficient emulsifying and dispersing effect cannot be achieved, and when the concentration of the polysaccharide exceeds 1 % by weight, improvement of the emulsification- dispersing effect can be achieved, but the viscosity of the agent increases to remarkably degrade the usability thereof.

The emulsification-dispersing agent of the present invention is prepared by applying a shearing force to the polysaccharide of the present invention with a melting device capable of applying a strong shearing force, such as a homogenizer, a disperser, and a dispermixer, into a particulate structure, and dissolving or dispersing the polysaccharide in water, whereby an aqueous solution (or dispersion liquid in water) of polysaccharide is obtained. The shearing force of the homogenizer, disperser, and dispermixer is determined to 0.5 m/s or more, preferably 0.9 m/s or more, at a tip speed thereof. When the shearing force is less than 0.5 m/s at a tip speed thereof, there may the cases where the polysaccharide of the present invention does not become a monoparticle sufficiently. And in order to improve the solubility and dispersing properties of the polysaccharide, the polysaccharide may be previously wetted with a lower alcohol such as methanol, ethanol, isopropyl alcohol, etc. prior to preparing of the emulsification-dispersing agent of the present invention.

Where the polysaccharide has a particulate structure, and, in particular, forms a monoparticle, the formation of the particulate structure is accelerated by adding urea to the solution of the polysaccharide. It is preferable that the content of urea ranges from 0.1 to 10 % by weight to the total amount of the cosmetic.

The method for producing a cosmetic in accordance with the present invention is the method of contacting an emulsification-dispersing agent composed of a polysaccharide having a particulate structure (polysaccharide of the present invention), and a component to be emulsification-dispersed, which is composed of an oily base material having a dielectric constant ranging from 1 (F/m) to 5 (F/m), and an inorganicity/organicity ratio ranging from 0 to 0.5, and/or a pigment with water, mixing together, emulsification-dispersing the same, thereby producing a cosmetic of which the thermal stability and the storage stability are improved. More specifically, by mixing the emulsification-dispersing agent composed of a polysaccharide having a particulate structure and the component to be emulsification-dispersed, which is composed of an oily base material having a dielectric constant ranging from 1 (F/m) to 5 (F/m), and an inorganicity/organicity ratio ranging from 0 to 0.5, and/or a pigment while agitating, the three-phase emulsification-dispersing system composed of "water phase (solvent)-intermediate layer containing a polysaccharide with a particulate structure-phase to be emulsification-dispersed" is formed, and by cooling while agitating, after aged, the cosmetic in accordance with the present invention can be obtained.

Since the polysaccharide to be used in the present invention is difficult to be dispersed in water, there is also the method of preparing an aqueous solution (or a dispersed liquid in water) of an emulsification-dispersing agent composed of a polysaccharide having a particulate structure by means of a homogenizer, disperser, dispermixer, etc., mixing and agitating a component to be emulsification-dispersed, which is composed an oily base material having a dielectric constant ranging from 1 (F/m) to 5 (F/m), and an inorganicity/organicity ratio ranging from 0 to 0.5, and/or a pigment, thereby forming a three-phase emulsification-dispersing system composed of "water phase (solvent)-intermediate layer containing a polysaccharide with a particulate structure-phase to be emulsification-dispersed", and cooling while agitating after aged. In either case, it is preferable to make the polysaccharide with a particulate structure into a monoparticle by applying a shearing force with a tip speed of 0.5m/s or more by means of a homogenizer, disperser, dispermixer, etc upon emulsification-dispersing.

The composition ratio of the polysaccharide of the present invention in the emulsification-dispersing agent may be arbitrarily determined in view of the content of the polysaccharide of the present invention in an objective cosmetic, and the required stability of the emulsification-dispersing properties for the cosmetic. For example, considering that the polysaccharide of the present invention is normally blended in an amount of 0.001 % by weight to 1 % by weight, preferably 0.01 % by weight to 0.5 % by weight, and more preferably 0.05 % by weight to 0.2 % by weight, relative to the total amount of the cosmetic, the composition ratio of the polysaccharide of the present invention in the emulsification-dispersing agent may be determined.

In addition, in the case of the method of previously dispersing the emulsification-dispersing agent containing the polysaccharide of the present invention in water, the concentration of the polysaccharide in the aqueous solution normally ranges from 0.01 % by weight to 8 % by weight, preferably 0.05 % by weight to 0.2 % by weight. In the case of the concentration of the polysaccharide being less than 0.01 % by weight, the amount of the polysaccharide becomes insufficient upon preparing the cosmetic, whereby there is a possibility that expected effects of the present invention are not obtained, whereas in the case of the concentration of the polysaccharide exceeding 0.8 % by weight, the aqueous solution of the polysaccharide becomes viscous so that handling properties may become inferior and the working efficiency may be lowered. In addition, upon mixing the emulsification-dispersing agent with the component to be emulsification-dispersed, the heating process may be performed in order to accelerate the emulsification-dispersing.

In the production of the cosmetic of the present invention, the polysaccharide contained in the emulsification-dispersing agent is made into a particulate structure by applying a shearing force with a homogenizer, disperser, dispermixer, etc. at a tip speed of 0.5m/s or more upon emulsification-dispersing, so as to surround a periphery of the component to be emulsification-dispersed, and adhere thereto into a layer configuration, whereby an intermediate layer is formed between water and the component to be emulsification-dispersed, and consequently, the three-phase emulsification-dispersing state is formed. In particular, it is preferable that the polysaccharide is made into a monoparticle.

Upon emulsification-dispersing, the mixing ratio of the emulsification-dispersing agent and the component to be emulsification-dispersed ranges from 1:50 through 1:1000, preferably 1:100 through 1:500 as a weight ratio of the polysaccharide adapted to form a particulate structure in the emulsification-dispersing agent to the component to be emulsification-dispersed. Outside this range, there may be the case where the emulsification-dispersing state of the present invention may not be obtained. In addition, it is preferable that, upon emulsification-dispersing, the average particle diameter of the polysaccharide of the present invention ranges from 8 nm to 500 nm. When the average particle diameter of the polysaccharide with a particulate structure is less than 8 nm, the attracting action due to the van der Waals' force becomes small, and consequently, there may be the case where the polysaccharide is difficult to adhere to the surface of the component to be emulsification-dispersed. In addition, when the average particle diameter is greater than 500 nm, there may be the case where a stable emulsion cannot be maintained.

In the conventional emulsification-dispersing method using surfactants, the surfactants form a monomolecule film on a surface of a periphery of a component to be emulsification-dispersed to lower surface energy, whereby emulsification-dispersing occurs to form an emulsion. As a result, this emulsion coalesces with another one due to thermal shock, and consequently, the storage stability is degraded. On the other hand, with the three-phase emulsifying method in accordance with the present invention, the surface energy hardly changes, and the oily base material as the component to be emulsification-dispersed is limited to that having the above-described specific dielectric constant and inorganicity/organicity ratio so that coalescence of emulsions is difficult to occur, and consequently, an emulsion which is high in thermal stability and storage stability is obtained.
The dielectric constant of the present invention can be measured, but is a well known value peculiar to substances, which is shown in The Chemical Handbook, etc. In addition, the inorganic value and the organic value are also well known values peculiar to substances, and can be obtained from "Technique of Emulsification and Solubilization" edited by Susumu Tsuji, Kougakutosho Ltd. (1976), which is shown above as Non patent document 1.

The cosmetic in accordance with the present invention includes many kinds of cosmetics according to their uses, and purified water, hot spring water, deep sea water, thickening agents, coloring agents, moisturizers, astringents, whitening agents, UV preventive agents, anti-inflammatory agents, skin (cell) activating agents, antibacterial agents, skin absorbing accelerating agents, carbonated agents, anti-oxidants, antiseptic agents, chelating agents, fade preventive agents, buffering agents, etc. may be further added arbitrarily according to demand. The present invention does not limit the blending of these various additives provided that the effects of the invention are not damaged.

Examples of the thickening agent include natural high polymers such as gum arabic, guar gum, karaya gum, carrageenan, pectin, fucoidan, tragant gum, locust bean gum, galactomannan, curdlan, gellant gum, fuco gel, casein, gelatine, starch, collagen, etc., semi-synthetic high polymers such as methyl cellulose, ethyl cellulose, methyl hydroxypropylcellulose, carboxymethylcellulose, hydroxymethylcellulose, hydroxypropylcellulose, sodium carboxymethylcellulose, alginic acid propylene glycol ester, etc., and synthetic high polymers such as polyvinyl alcohol, polyvinylpyrrolidone, carboxyvinyl polymer, polyacrylic acid, sodium polyacrylate, potassium polyacrylate, polyethyleneoxide, ethyleneoxidepropylene oxide copolymer, etc. Inorganic minerals such as bentonite, laponite, hectorite, etc. may be used together.

Examples of the moisturizer (component) include alkali simple spring water, deep sea water, mucopolysaccharides such as hyaluronic acid, chondroitin sulphate, dermatan sulfate, heparan sulfate, heparin, keratan sulfate, etc. or salts thereof, proteins such as collagen, elastin, keratin, derivatives thereof, and salts thereof, phospholipids derived from soybean and egg, glycolipids, ceramide, mucin, phoney, erythritol, maltose, maltitol, xylitol, xylose, pentaerythritol, fructose, dextrine and derivatives thereof, saccharides such as mannitol, sorbitol, inositol, trehalose, glucose, etc., urea, asparagine, aspartic acid, alanine, arginine, isoleucine, orthinine, glutamine, glycine, glutamic acid, derivatives thereorf, and salts thereof, cysteine, cystine, citrulline, threonine, cerine, tyrosine, tryptophan, theanine, valine, histidine, hydroxylysine, hydroxyproline, pyrrolidone carbonate, salts thereof, amino acids such as proline, phenylalanine, methionine, lysine, derivaties thereof and salts thereof, D-panthenol, and plant extract liquids. Examples of the plant extract liquid include avocado extract, almond oil, carob extract, rice plant extract, strawberry extract, anise extract, pale red hollyhock extract, coptis extract, olive oil, lamium album extract, cacao butter, wild oat extract, ivy extract, sasa albo-marginata extract, gardenia extract, grapefruit extract, geranium thumbergii extract, great yellow gentian extract, burdock extract, tree peony vine extract, sesame extract, cactus extract, saponaria officinalis extract, ginger extract, rehmannia root extract, shear butter, spiraea extract, cnidium officinale extract, mallow extract, thymus vulgaris extract, camellia extract, corn extract, cordyceps sinensis extract, tormentila extract, houttuynia extract, ophiopogon tuber extract, lupinus perennis extract, hamamelis extract, mint extract, mentha viridis extract, peppermint extract, parsley extract, rose extract, sunflower extract, hinoki extract, looffah extract, prune extract, bucher's broom extract, borage oil, peony extract, jojoba oil, tilia miqueliana extracts, hop extract, pine extract, marronnier extract, macadamia nut oil, quince extracts, gromwell extracts, meadowfoam oil, melissa extracts, cornflower extracts, lily extracts, citron extracts, lime extracts, lavandula vera extract, gertianas scabra extract, sanguisorba extracts, apple extracts, etc. Yeast metabolite, yeast extraction extract, rice fermentation extract, fermented rice bran extract, euglena extract, lactic fermentation thing of raw milk and skim milk, trehalose derivatives thereof can be used. Examples of the alcohol and polyalcohol include natural alcohols such as ethanol, isopropanol, lauryl alcohol, cetanol, stearyl alcohol, oleyl alcohol, lanolin alcohol, cholesterol, phytosterol, etc., and synthetic alcohols such as 2-hexyl decanol, isostearyl alcohol, 2-octyl dodecanol, etc., ethylene oxide, ethylene glycol, diethylene glycol, triethylene glycol, ethylene glycol monoethyl ether, ethylene glycol monobutyl ether, diethylene glycol monomethyl ether, diethylene glycol monoethyl ether, polyethylene glycol, propylene oxide, propylene glycol, polypropylene glycol, 1,3- butylene glyc ol, glycerin, pentaerythritol, sorbitol, mannitol, etc. One or more of these moisturizers can be selected properly and blended, and the blending amount depends on the kind of the moisturizers, but, normally, ranges from 0.5 to 20 %.

Examples of the astringent (component) include zinc phenolsulfonate, sodium phenolsulfonate, and plant extracts. Examples of the plant extracts include arnica, hawthorn, cinchona, salvia, tilia miqueliana, ginseng, juniperus communis, rosemary, hypericum, ginkgo tree, melissa, ononis spinosa, marronnier, swertia japonica, garlic, chamomile, thyme, mint, nettle, red pepper, ginger, hop, western horse chestnut, lavandula vera, carrot, leaf mustard, cinnamomum cassia, pine, cnidium officinale, elder, ostericum sieboldii, scopolia, peony, myrica, houttuynia, oryza sativa bone, bitter persimmon, calendula officinalis, corn poppy, gertiana scabra, grape, glehnia, orange, citron, iris, japanese summer orange, hamamelis, sweet dover, anise, japanese pepper, peony, eucalyptus, artemisia vulgaris indica, isodon japonicus, rice, sophora angustifolia, bread, dove, leaf of the walnut, scutellaria root, sage, tuberous roots of polygonum multiflorum, coptidis rhizoma, phellodendri cortex, scuttellaria baicalensis georgi, houttuyniae herba, aurantii nobilis pericarpium, codonopsitis radix, propolis, alisma rhizome, tannin, birch wood tar, royal jelly, yeast extract, etc.
One or more of these materials can be used in combination as the astringents. Normally, the amount thereof ranges from 0.001 to 5 % by weight, preferably, from 0.01 to 3 % by weight, of the total amount of the cosmetic.

Examples of the whitening agent (component) include tyrosinase inhibitor, endothelin antagonist, α -MSH inhibitor, glabridin, glablene, liquilitin, isoliquilitin, ellagic acid, ellagic acid salts, ellagic acid derivatives, kojic acid, kojic acid salts, kojic acid derivatives, arbutin, arubutin salts, arubutin derivatives, cysteine, cysteine salts, cysteine derivatives, vitamin C such as ascorbic acid, sodium ascorbate, ascorbyl stearate, ascorbyl palmitate, ascorbyl di-palmitate, magnesium ascorbyl-2-phosphate, vitamin C salts, vitamin C derivatives, glutathione, glutathione salts, glutathione derivatives, resorcin, resorcin salts, resorcin derivatives, rucinol, neo agarobiose, agarose oligosaccharide, and plant extracts. Examples of the plant extract include asparagus extract, althaea officinalis extract, bistorta major extract, artemisiae capillaris flos extract, pea extract, dog rose fruit extract, scutellaria root extract, ononis spinosa extract, seaweed extract, firethorm extract, licorice extract, raspberry extract, sophora root extract, brown sugar extract, millettja reticulate extract, acanthopanacis cortex extract, wheat germ extract, asiasari radix extract, hawthorn extract, nomame herba extract, peony extract, lily extract, inula flower extract, mulberry bark extract, soybean extract, placenta extract, aralia elata extract, tea extract, angelica acutiloba extract, moleasses extract, rosa polyantha extract, ampelopsis japonica extract, grape seed extract, beech extract, flor de manita extract, hop extract, rosa rugosae flos extract, chaenomelis fructus extract, saxifraga extract, coicis semen extract, rakanka extract, etc. One or more of these materials can be used properly. Normally, the blending amount of the whitening agents ranges from 0.01 to 10 %. Where the plant extracts, etc. are used as extracted liquids, the above blending amount is the amount converted into a dried solid extract.

Ultraviolet rays care agents (components) include organic compounds-based ultraviolet rays absorbents and inorganic compounds-based ultraviolet rays scattering agents. Examples of ultraviolet absorbents include para-aminobenzoic acid-based ultraviolet absorbents, cinnamic acid-based ultraviolet absorbents, salicylic acid-based ultraviolet rays absorbents, benzophenone-based ultraviolet rays absorbents, etc. One or more of these absorbents is blended. Examples of the para-aminobenzoic acid-based ultraviolet rays absorbent include para-aminobenzoic acid, glyceryl para-aminobenzoate, ethyl dihydro propyl para-aminobenzoate, amyl para dimethyl para-aminobenzoate, octyl para methyl para-aminobenzoate, ethyl para-aminobenzoate, isobutyl para-aminobenzoate, etc. Examples of the cinnamic acid-based ultraviolet absorbents include isopropyl para methoxy cinnamate, diisopropyl cinnamic acid ester, octyl methoxy cinnamate, di-para methoxy cinnamate mono 2-ethylhexanoic acid glyceryl, etc., examples of the salicylic acid-based ultraviolet rays absorbents include homo menthyl salicylate, octyl salicylate, phenyl salicylate, salicylic acid triethanolamine, amyl salicylate, benzil salicylate, p-tert butylphenyl salicylate, ethylene glycol salicylate, salicylic acid, etc. Examples of the benzophenone-based ultraviolet rays absorbents include dihydroxybenzophenone, tetrahydroxy benzophenone, oxybenzone, oxybenzone sulfonic acid, sodium hydroxy methoxybenzo phenon sulphonate, dihydroxy dimethoxybenzophenone, 2-hydroxy chlorobenzophenone, dioxybenzone, sodium dihydroxy dimethoxybenzophenone disulphonate, 2-hydroxy-4-methoxy-4' methylbenzophenone, octabenzone, etc. In addition, urocanic acid, ethyl urocanate, 4-tert-4'-methoxy dibenzoyl-methane, 2-(2'-hydroxy-5'-methylphenyl)benzotriazole, anthranilic acid, etc. can be used. Examples of the inorganic compound to be used as the ultraviolet rays scattering agent include titanium dioxide, zinc oxide, cerium oxide, zirconium oxide, iron oxide, etc.

Examples of te antiinflammatory agent (component) include zinc oxide, sulfur, derivatives of sulfur, glycyrrhizin acid, derivatives and salts of glycyrrhizin acid, such as di-potassium glycyrrhigate and mono-ammonium glycyrrhigate, glycyrrhetic acid, derivatives and salts of glycyrrhetic acid such as β-glycyrrhetic acid, stearyl glycyrrhetinate, disodium 3-succinyloxyglycyrrhetinate, etc, tranexamic acid, chondroitin sulfate, mefenamic acid, phenylbutazone, indometacin, ibuprofen, ketoprofen, allantoin, guaiazulene, and derivatives and salts thereof, various kinds of microbes, animal extracts, plant extracts, etc.

Examples of the skin (cell) activating agent (component) include deoxyribonucleic acid, salts of deoxyribonucleic acid, adenylic acid derivatives such as adenosine triphosphate, adenosine phosphate, salts thereof, ribonucleic acid, salts thereof, cyclic AMP, cyclic GMP, flavin adenine dinucleotide, guanine, adenine, cytosine, thymine, xanthine and derivatives threof, caffeine, theophylline and salts thereof, retinol, retinol derivatives such as retinol palmitate, retinol acetate, etc., retinal, retinal derivatives such as dehydroretinal, etc., carotene, vitamin A such as carotenoid, etc., thiamine, thiamine salts such as thiamine hydrochloride, thiamine sulfate, etc., riboflavin, riboflavin salts such as riboflavin acetate, etc., pyridoxine, pyridoxine salts such as pyridoxine hydrochloride, pyridoxine di-octanoate, etc, flavin adenine dinucleotide, cyanocobalamin, folic acids, nicotinic acid, nicotinic acid derivatives such as nicotinamide, nicotinic acid benzil, etc., vitamin B such as choline, etc., γ - linolenic acid and derivatives thereof, eicosapentaenoic acid and derivatives thereof, estradiol and derivatives and salts thereof, organic acids such as glycolic acid, succinic acid, lactic acid, salicylic acid, etc. and derivatives and salts thereof.

Examples of the antibacterial agent (component) include benzoic acid, sodium benzoate, carbolic acid, sorbic acid, potassium sorbate, para oxybenzoic acid ester, para chloro metacresol, hexachlorophene, benzalkonium chloride, chlorhexidine chloride, trichlorocarbanilide, quaternum, bis (2-pyridylthio-1-oxide)zinc, phenoxyethanol, thianthol, isopropyl methylphenol, etc.

Examples of the antioxidant (component) include vitamin A such as retinol, dehydroretinol, retinyl acetate, retinyl palmitate, retinal, retinoic acid, vitamin A oil, derivatives and salts thereof, carotenoids such as α -carotene, β - carotene, γ -carotene, cryptoxanthin, astaxanthin, fucoxanthin, etc. and derivatives and salts thereof, vitamin B such as pyridoxine, pyridoxal, pyridoxal-5-phosphate ester, pyridoxamine, etc., and derivatives and salts thereof, vitamin C such as ascorbic acid, sodium ascorbate, ascorbyl stearate, ascorbyl palmitate, ascorbyl dipalmitate, ascorbic acid magnesium phosphate, etc. and derivatives and salts thereof, vitamin D such as ergocalciferol, cholecalciferol, 1,2,5-dihydroxy-cholecalciferol, etc., derivatives and salts thereof, vitamin E such as α -tocopherol, β -tocopherol, γ -tocopherol, δ -tocopherol, α - tocotrienol, β -tocotrienol, γ -tocotrienol, δ -tocotrienol, tocopherol acetate, tocopherol nicotinate, etc., derivatives and salts thereof, tororocks(water-soluble derivatives of vitamine E), derivatives and salts thereof, dihydroxytoluene, butylhydroxytoluene, butylhydroxyanisol, dibutylhydroxytoluene, α -lipoic acid, dehydrolipoic acid, glutathione, derivatives and salts thereof, uric acid, erythorbic acid, derivatives and salts thereof such as sodium erythorbate,etc., gallic acid, derivatives and salts thereof such as propyl gallate,etc., rutin, derivatives and salts thereof such as α -glycosyl-rutin, etc., tryptophan, derivatives and salts thereof, histidine, derivatives and salts thereof, derivatives and salts of cysteine such as N-acetylcysteine, N-acetylhomocysteine, N-octanoilcysteine, N-acetylcysteinemethylester, etc. derivatives and salts of cystine such as N, N'-diacetylcystinedimethylester, N, N'-dioctanoilcystinedimethylester, N, N'-dioctanoilhomocystinedimethylester, etc., carnosine, derivatives and salts thereof, homocarnosine, derivatives and salts thereof, anserine, derivatives and salts thereof, calcinine, derivatives and salts thereof, derivatives and salts of dipeptide or tripeptide containing histidine and/or triptophan and/or histamine, flavonoids such as flavanone, flavone, anthocyanin, anthocyanidin, flavonol, quelcitron, myricetin, phycetin, hamamelis tannin, catechin, epicatechin, gallocatechin, epigalocatechin, epicatechingallate, epigallocatechingallate, etc., tannic acid, caffeic acid, ferulic acid, protocatechuic acid, chalcone, oryzanol, carnot sole, sesamole, sesamin, sesamolein, zingerone, curcumin, tetrahydro curcumin, chlobamide, deoxychlobamide, shoga-ol, capsaicin, vanillylamide, ellagic acid, bromphenol, flavoglassine, melanoidin, riboflavin, riboflavin butylester, flavinmononucleotide, flavin adenine nucleotide, ubiquinone, ubiquinol, mannitol, bilirubin, cholesterol, ebselen, selenomethionine, ceruloplasmin, transferrin, lactoferrin, albumin, bilirubin superoxide dismutase, catalase, glutathione peroxidase, metallothionein, O-phosphono-pyrid oxylidene rhodamine, N-(2-hydroxybenzyl) amino acid, derivatives and salts thereof, N-(4-pyridoxyl methylene) amino acid, derivatives and salts thereof, which are disclosed in U.S.P. No.5,594,012, etc. The content of the antioxidant depends on the kind thereof so as not to be determined equally. But, normally, the content thereof ranges from 0.01 to 10 %. Where the plant extract, etc. are used as extracted liquids, the above-described content is the amount converted into a dried solid extract.

Examples of the perfume (components) include natural perfumes and synthetic perfumes. Examples of the natural perfume include natural plant perfume such as rose oil, jasmine oil, neroli oil, lavender oil, tuberose oil, ylang ylang oil, clary sage oil, clove oil, peppermint oil, geranium oil, pachouli oil, sandlwood oil, cinnamon oil, coriander oil, nutmeg oil, pine oil, vanilla oil, balsam peru oil, banana oil, apple oil, fennel oil, tonca beans oil, pepper oil, lemon oil, orange oil, bergamot oil, opopanax oil, vetiver oil, iris oil, oakmoss oil, anise oil, bois de rose oil, etc., and natural animal perfumery such as musk oil, civet oil, castoreum oil, ambergris oil, etc.

Examples of the synthetic perfume include hydrocarbons such as limonene, β -caryophylene, etc., alcohols such as cis-3-hexenol, linalool, farnesol, β -phenylethylalcohol, geraniol, citronellol, terpineol, menthol, santalol, bacdanol, puramanol, etc., aldehydes such as lyranol, lilial, 2 , 6-nonadienal, citral, α -hexyl cinnamic aldehyde, etc., ketones such as β -ionone, 1-carvone, cyclopenta decanone, damascone, methyl ionone, irone, ISO E-super, acetyl cedrene, muscone, etc., esters such as benzyl acetate, methyl dihydrojasmonate, methyl jasmonate, linalyl acetate, benzyl benzoate, etc., lactones such as γ -undecalactone, jasmine lactone, cyclopentadecanolide, ethylene brassylate, etc., oxides such as galactsolid, ambroxane, rose oxide, etc., phenols such as eugenol, etc., nitrogenous compounds such as indole, etc., acetals such as phenylacetaldehydedimethylacetal, etc., Schiff bases such as aurnriol, etc. Normally, only one kind of perfume is not used, but a plurality of perfumes are used in combination as a compound perfume according to objects thereof.

Examples of the organic solvent (component) include ethanol, acetone, ethyl acetate, butyl acetate, 1,3-butylene glycol, ethylene glycol, diethylene glycol, triethylene glycol, polyethylene glycol, propylene glycol, dipropylene glycol, glycerin, butanol, propanol, etc.

Examples of the sequestering agent and the antiseptic agent include hidroxyethane diphosphonic acid salts, phenacetine, EDTA & salts thereof, parapenes, stannates, etc., and examples of the high molecular compound include poly(dimethyl allyl ammonium halide)type cationic polymer, tallowyl amine condensation product type cationic polymer obtained from polyethylene glycol, epichlorohydrin, propylene amine and tallow fatty acid, cocoyl amine condensation product type cationic polymer obtained from polyethylene glycol, epichlorohydrin, propylene amine and coconut oil fatty acid, copoymer type cationic polymer of vinyl pyrrolidone and dimethyl aminomethaacrylate, guaternary nitrogen-containing cellulose ether type cationic polymber, etc.
Examples of the pH adjusting agent include organic acids such as citric acid, malic acid, acetic acid, lactic acid, oxalic acid, tartaric acid, formic acid, levulinic acid, etc. and inorganic acids such as phosphoric acid, hydrochloric acid, etc.

### EMBODIMENT

Hereinafter, embodiments of the present invention will be explained.
Polysaccharides (A-1) through (A-8) to be used in the embodiments of the present invention are as follows:
(1) Polysaccharide (A-1): a product polysaccharide of a microorganism of Alcaligenes latus strain B-16 (rough product)
40.0 g of glucose [Wako Pure chemical Industries, Ltd., reagent], 4.0 g of dipotassium hydrogen phosphate [Wako Pure chemical Industries, Ltd., reagent], 2.0 g of potassium dihydrogen phosphate [Wako Pure chemical industries, Ltd., reagent], 0.1 g of sodium chloride [Wako Pure chemical Industries, Ltd., reagent], 0.2 g of magnesium sulfate [Wako Pure chemical Industries, Ltd., reagent], 1.0 g of potassium nitrate [Wako Pure chemical Industries, Ltd., reagent], and 1.5 g of yeast extract [OXOID CO. Ltd.] were dissolved in an ion exchange water, and an obtained aqueous solution was adjusted to a pH of 6.5 using sodium hydroxide or sulphuric acid so that the total volume is 1 liter. 150 mL of the obtained aqueous solution was transferred into a 500 mL conical flask and sterilized by autoclaving at 121 °C for 15 minutes. Then, the temperature of the solution was lowered to room temperature, and Alcaligenes latus strain B-16 (FERM BP-2015) was inoculated in the solution in the flask. And the solution was subjected to shaking culture at 30 °C for 6 days(180 rpm). After cultivation, about three volumes of isopropyl alcohol was added thereto and agitated for mixing them. A resultant precipitated agglomeration was filtered, recovered and dried under a reduced pressure to obtain polysaccharides of a product of a microorganism of Alcaligenes latus strain B-16 (A-1). The obtained polysaccharide includes a polysaccharide composed of fucose, glucose, glucuronic acid and rhamnose in a molar ratio of 1:2:1:1, as a main component, and another polysaccharide composed of fucose and mannose in a molar ratio of 1:1 in the weight ratio of 7:1. The polysaccharide was hydrolyzed with sulfuric acid, and resultant constituting monosaccharides were analyzed with a high speed liquid chromatography (HPLC).

(2) Polysaccharide (A-2): purified product of the above-described Polysaccharide (A-1)
An aqueous solution of 0.5 wt% of polysaccharide: A-1 was prepared, and an aqueous solution of sodium hydroxide was added thereto to a pH of 12. The obtained aqueous solution was processed using columns of ion exchange resin "DIAON HPA-75(OH-)(trade name)"(manufactured by Nippon Rensui, CO.) at 8Ru or less, and filtered with a filtration auxiliary "Radiolight RL700" and a membrane filter of 5 µ m to remove proteins, nucleic acids, and microbes. After the filtered liquid was adjusted to a pH of 7 with dilute hydrochloric acid, the pressure of the liquid was reduced, and the liquid was concentrated. Then the polysaccharides were precipitated by using acetone, and washed with ten volumes of acetone to obtain polysaccharide (A-2) composed of fucose, glucose, glucuronic acid and rhamnose in the ratio of 1:2:1:1 and having a high molecular weight of fifty million.

(3) Polysaccharide (A-3): Alcasealan (manufactured by HAKUTO CO., LTD.)
(4) Polysaccharide (A-4), Polysaccharide (A-5): product polysaccharides of microorganisms of sphingomonas trueperi SPH-011and SPH-012 (rough products)
50 L of a medium having the later-describing composition is put into a fermentation tank of 90L, which is manufactured by Marubishi engineering co., Ltd., and after sterilized, sphingomonas trueperi SPH-011 (FERM BP-08582) or SPH-012 (FERM BP-08579) was respectively taken, and cultured. The fermentation tank was agitated using a turbine agitating fin as an agitating fin thereof in the range of 700 rpm through 800 rpm, the amount of air flow was determined in the range of 1 vvm through 2vvm. pH was controlled to the range of 6.5 ± 0.4 with an aqueous solution of NaOH· 1 N. And the culturing temperature was controlled at 30°C ± 0.2. The culture was performed for six days. After the culturing step is finished, about three volumes of isopropyl alcohol was added to the cultured substance and agitated for mixing them. A resultant precipitated agglomeration was filtered, recovered and dried under a reduced pressure to obtain a product polysaccharide (A-4) of a microorganism of sphingomonas trueperi SPH-011 and a product polysaccharide (A-5) of a microorganism of sphingomonas trueperi SPH-012. The polysaccharide (A-4) includes a polysaccharide composed of fucose, glucose, glucuronic acid and rhamnose in a molar ratio of 1:2:1:1, as a main component, and another polysaccharide composed of fucose and mannose in a molar ratio of 2 :1. And the polysaccharide (A-5) includes a polysaccharide composed of fucose, glucose, glucuronic acid and rhamnose in a molar ratio of 1:2:1:1, as a main component, and another polysaccharide composed of fucose and mannose in a molar ratio of 1:1. And the polysaccharide was hydrolyzed with sulfuric acid, and resultant constituent monosaccharides were analyzed with a high speed liquid chromatography (HPLC).

### <Composition of a medium>

| | |
|---|---|
| glucose | [Wako Pure chemical Industries, Ltd.] 4.00 g |
| dipotassium hydrogen phosphate | [Wako Pure chemical Industries, Ltd] 0.40 g |
| potassium dihydrogen phosphate | [Wako Pure chemical Industries, Ltd] 0.20 g |
| sodium chloride | [Wako Pure chemical Industries, Ltd] 0.01 g |
| magnesium sulfate | [Wako Pure chemical Industries, Ltd.] 0.02 g |
| potassium nitrate | [Wako Pure chemical Industries, Ltd.] 0.10 g |
| yeast extract HY YEAST412 | [Sigma Corporation] 0.15 g |

(5) Polysaccharides (A-6), (A-7): product polysaccharides of microorganisms of sphingomonas trueperi SPH-011 and SPH-012 (purified products)
Sodium hydroxide was added to aqueous solutions of 0.5 wt% of polysaccharides (A-4) and (A-5) so as to have a concentration of 0.02 % by weight, and agitated for one night, thereby dispersing the polysaccharides. In addition, the aqueous solutions were heated under the condition of 121°C and 10 minutes, thereby dissolving the polysaccharides. Next, centrifugation was performed (40, OOOG, 40 minutes). The removal of bacteria was confirmed based on the transparency of a top clear part of the aqueous solutions. Next, by filtering the same with the above-described membrane filter system, and filtered residue was obtained. About 100 volumes of pure water was added again to this filtered residue, and, after agitated, filtering operation is performed again. This operation was repeated five times and insoluble components were demineralized. The insoluble components in a gel state, which had been dehydrated with the membrane filter system to some degree, were subjected to the decompressing and drying operation at normal temperature, and consequently, a product polysaccharide (A-6) of a microorganism of sphingomonas trueperi SPH-011 and a product polysaccharide (A-7) (purified product) of a microorganism of sphingomonas trueperi SPH-012 (purified product) were obtained.

(6) Polysaccharide (A-8) used in Comparative example: xanthan gum (KELZAN AX manufactured by CP Kelco)

(7) Substance to be emulsification-dispersed
The kind, dielectric constant, organicity, and inorganicity of substances to be emulsification-dispersed (B-1) through (B-22), which were used in Embodiments of the present invention, were shown in Table 1.

**[Table 1]**

| No. | Substance to be emulsification-dispersed | Dielectric constant (F/m) | Organicity | Inorganiciy | Inorganicity/ organicity ratio |
|---|---|---|---|---|---|
| B-1 | tetradecanol (reagent; Kanto Chemical Co., Inc.) | 4.72 | 280 | 100 | 0.36 |
| B-2 | hexadecanol (reagent; Kanto Chemical Co., Inc.) | 3.82 | 320 | 100 | 0.31 |
| B-3 | octadecanol (reagent; Kanto Chemical Co., Inc.) | 3.42 | 360 | 100 | 0.28 |
| B-4 | palmitic acid (reagent; Kanto Chemical Co., Inc.) | 2.30 | 320 | 150 | 0.47 |
| B-5 | stearic acid (reagent; Kanto Chemical Co., Inc.) | 2.29 | 360 | 150 | 0.42 |
| B-6 | oleic acid (reagent; Kanto Chemical Co., Inc.) | 2.46 | 360 | 152 | 0.42 |
| B-7 | cyclohexane (reagent; Kanto Chemical Co., Inc.) | 2.00 | 360 | 152 | 0.42 |
| B-8 | 1-tetradecanol (reagent; Kanto Chemical Co., Inc.) | 4.72 | 280 | 110 | 0.39 |
| B-9 | ethyl oleate (reagent; Kanto Chemical Co., Inc.) | 3.17 | 400 | 60 | 0.15 |
| B-10 | butyl oleate (reagent; Kanto Chemical Co., Inc.) | 4.00 | 440 | 62 | 0.14 |
| B-11 | 1-bromooctane (reagent; Kanto Chemical Co., Inc.) | 5.00 | 220 | 10 | 0.45 |
| B 12 | hexane (reagent; Kanto Chemical Co., Inc.) | 1.89 | 120 | 0 | 0.00 |
| B-13 | octane (reagent; Kanto Chemical Co., Inc.) | 1,95 | 160 | 0 | 0.00 |
| B-14 | decane (reagent; Kanto Chemical Co., Inc.) | 1.99 | 200 | 0 | 0.00 |
| B-15 | dodecane (reagent; Kanto Chemical Co., Inc.) | 2.02 | 240 | 0 | 0.00 |
| B-16 | hexanol (reagent; Kanto Chemical Co., Inc.) | 13.30 | 120 | 100 | 0.83 |
| B-17 | octanol(reagent; Kanto Chemical Co., Inc.) | 10.30 | 160 | 100 | 0.63 |
| B-18 | hexanoic acid (reagent; Kanto Chemical Co., Inc.) | 2.63 | 120 | 150 | 1.25 |
| B-19 | octanoic acid (reagent; Kanto Chemical Co., Inc.) | 2.54 | 160 | 150 | 0.94 |
| B-20 | cyclohexanol (reagent; Kanto Chemical Co., Inc.) | 15.00 | 120 | 110 | 0.92 |

The emulsification-dispersing agent and the component to be emulsification-dispersed, which were adapted to be used in Embodiments of the present invention, were subjected to an emulsification-dispersing test, as follows.

### (Emulsification-dispersing test 1)

Polysaccharide (A-1) was put into water to prepare a water-dispersed liquid containing 0.05 % by weight of polysaccharide (A-1). Then, by applying a shearing force with a homogenizer (manufactured by Tokushukika Co., Ltd.) at 8000 rpm, the water-dispersed liquid containing 0.05 % by weight of polysaccharide (A-1) was agitated for 20 minutes, thereby preparing a polysaccharide (A-1)-dispersed liquid (an aqueous solution containing 0.05 % by weight of polysaccharide (A-1)). 30 mL of tetradecanol (B-1) as the substance to be emulsification-dispersed was added to 100 mL of the polysaccharide (A-1)-dispersed liquid while agitating the same with a homogenizer at 8000rpm, and a resultant liquid was further agitated for 1 minute to be emulsification-dispersed. The obtained emulsification-dispersed substance was placed in each of thermostatic chambers of room temperature and 50°C, and, after 1 week and three months, the long-term stability of the emulsification-dispersed substance was evaluated by eyes, as follows:
○ : no separation in emulsion
△ : concentration difference is observed between upper part and lower part
× : separation of water and oil is observed

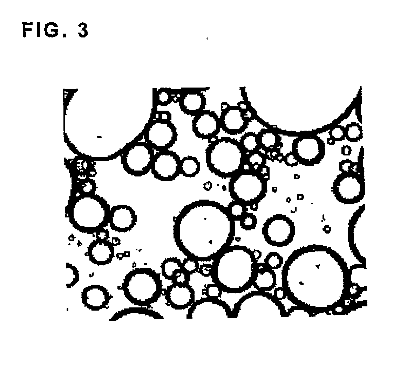

Similarly, emulsification-dispersed substances were prepared from various substances to be emulsification-dispersed, which were shown in Table 1, and the long-term stability thereof was evaluated. The evaluation results are shown in Table 2. In order to confirm the three-phase emulsification, one drop of the emulsification-dispersed substance was taken, and a liquid drop in an emulsification-dispersed state was observed with an optical microscope, and it was judged that the three-phase emulsification was maintained stably, if it was confirmed that each oil drop deforms and exists stably so as not to coalesce each other, in spite of oil drops contacting and pushing each other, as shown in FIG. 3 (in the emulsification-dispersing with surfactants, oil drops coalesce each other upon contacting each other).

**[Table 2]**

| Example | No | Polysaccharide | Substance to be emulsification-disperse | Stability of emulsification-dispersed substance with age | | |
|---|---|---|---|---|---|---|
| | | | | Room temper ature 1 week | Room temperat ure 3 months | 50°C 3 months |
| Embodiment | 1 | A-3 | B-1: B-2=1:1 | ○ | ○ | ○ |
| | 2 | | B-1: B-3=1:1 | ○ | ○ | ○ |
| | 3 | | B-1: B-4=1:1 | ○ | ○ | ○ |
| | 4 | | B-1: B-5=1:1 | ○ | ○ | ○ |
| | 5 | | B-1: B-6=1:1 | ○ | ○ | ○ |
| | 6 | | B-1: B-7=1:1 | ○ | ○ | ○ |
| | 7 | | B-1: B-8=1:1 | ○ | ○ | ○ |
| | 8 | | B-1: B-9=9:1 | ○ | ○ | ○ |
| | 9 | | B-1: B-10=1:1 | ○ | ○ | ○ |
| | 10 | | B-1: B-11=1:1 | ○ | ○ | ○ |
| | 11 | | B-1: B-12=1:1 | ○ | ○ | ○ |
| | 12 | | B-1: B-13=1:1 | ○ | ○ | ○ |
| | 13 | | B-1: B-14=1:1 | ○ | ○ | ○ |
| | 14 | | B-1: B-15=1:1 | ○ | ○ | ○ |
| | 15 | | B-2: B-8=1:1 | ○ | ○ | ○ |
| | 16 | | B-3: B-4=1:1 | ○ | ○ | ○ |
| | 17 | | B-4: B-9=1:1 | ○ | ○ | ○ |
| | 18 | | B-1: B-15=1:1 | ○ | ○ | ○ |
| | 19 | | B-1: B-5:B-10=1:1:1 | ○ | ○ | ○ |
| | 20 | A-1 | B-1: B-5:B-10=1:1:1 | ○ | ○ | ○ |
| | 21 | A-2 | | ○ | ○ | ○ |
| | 22 | A-4 | | ○ | ○ | ○ |
| | 23 | A-5 | | ○ | ○ | ○ |
| | 24 | A-6 | | ○ | ○ | ○ |
| | 25 | A-7 | | ○ | ○ | ○ |
| | 26 | A-8:A-3=1:1 | | ○ | ○ | ○ |
| | 27 | A-6:A-3=1:1 | | ○ | ○ | ○ |
| | 28 | A-7:A-3=1:1 | | ○ | ○ | ○ |
| | 29 | A-7:A-8:A-3=1:1:1 | | ○ | ○ | ○ |
| | 30 | A-8:A-3=1:1 | B-1: B-4=1:1 | ○ | ○ | ○ |
| Comparative example | 1 | A-3 | 8-1: B-16=1:1 | × | × | × |
| | 2 | | B-1: B-17=1:1 | × | × | × |
| | 3 | | B-1: B-18=1:1 | × | × | × |
| | 4 | | B-1: B-19=1:1 | × | × | × |
| | 5 | | B-1: B-20=1:1 | × | × | × |
| | 6 | | B-16: B-20=1:1 | × | × | × |

It has been clarified that the emulsification-dispersed substance obtained from the emulsification-dispersing agent mainly containing the polysaccharide used in the present invention, and two or more kinds of components to be emulsification-dispersed (oily base materials), each having a dielectric constant which ranges from 1 (F/m) through 5(F/m), and an inorganiciy/organicity ratio which ranges from 0 to 0.5, maintains stability at 50°C over three months even when the concentration of the emulsification-dispersing agent is as low as 0.05 % by weight so as to exhibit excellent stability in emulsification-dispersing, as compared with the comparative examples 1 through 8, in which such a component to be emulsification-dispersed (oily base materials) as not to have the dielectric constant ranging from 1(F/m) through 5(F/m), and an inorganiciy/organicity ratio ranging from 0 to 0.5 is blended or a part thereof is contained.

Hereinafter, the preparation of the cosmetic of the present invention, and evaluation results will be explained.
Embodiments 31 through 37 are preparations of milky lotions 1 through 7, Embodiments 38 through 43 are preparations of creams 1 through 6 and Embodiments 44 through 45 are preparations of sunscreens 1 through 2. Comparative examples 7 through 10 are preparations of milky lotions 8 through 9, cream 7 and sunscreen 3.

### (Embodiment 31: milky lotion 1)

The composition of milky lotion 1 is shown in Table 3.

**[Table 3]**

| Section | Components (trade name, maker) | % by weight |
|---|---|---|
| a | glycerin (glycerin S, Sakamoto Yakuhin Kogyo Co., Ltd.) | 5.00 |
| | diglycerin (diglycerin 801, Sakamoto Yakuhin Kogyo Co., Ltd.) | 2.00 |
| | 1, 3-butylene glycol (1, 3BG, DAICEL CHEMICAL INDUSTRIES, LTD.) | 7.00 |
| | 1-(2-ethyl hexyl)glycol ether (sencibaSC50, SEIWA KASEI Co., Ltd.) | 0.30 |
| | 1,2-pentandiol (diolPD, Nikko Chemicals Co., Ltd.) | 3.00 |
| | POE methyl glucoside (MagbioburaidoMG-20E, NOF CORPORATION) | 0.50 |
| b | tetradecanol (B-1) | 4.00 |
| | hexadecanol (B-2) | 4.00 |
| | stearic acid (B-5) | 4.00 |
| | natural vitamin E (Riken oil E700, Riken Vitamin co., Ltd.) | 0.01 |
| c | polysaccharide (A-3) | 0.05 |
| | purified water | balance |

The preparation method of milky lotion 1 is as follows.
1.Polysaccharide (A-3) of Section c was heated to 80°C, and pre-dispersed in water using a disperser (Dispersion liquid 1).
2. Components of Section a were respectively weighed, mixed with the dispersion liquid 1 homogeneously, and dissolved by heating at 80°C(Dispersion liquid 2).
3. Components of Section b were respectively weighed, and dissolved by heating at 80°C (Mixture liquid 1)
4. The mixture liquid 1 was gradually added to the dispersion liquid 2 while agitating with a homogenizer (or homomixer) at 8000 rpm. After added, a resultant mixture was further agitated for 10 minutes, and cooled to room temperature, whereby milky lotion 1 (Embodiment 31) was obtained.

### (Embodiment 32: milky lotion 2)

By replacing the tetradecanol (B-1) of Section b of Embodiment 31 with the same amount of octadecanol (B-3), and replacing the stearic acid (B-5) with the same amount of palmitic acid (B-4), milky lotion 3 (Embodiment 33) was obtained.

### (Embodiment 33, milky lotion 3)

By replacing the tetradecanol (B-1) of Section b of Embodiment 31 with the same amount of 1-tetradecanol (B-8), and replacing the stearic acid (B-5) with the same amount of oleic acid (B-6), milky lotion 3 (Embodiment 33) was obtained.

### (Embodiment 34, milky lotion 4)

By replacing the tetradecanol (B-1) of Section b of Embodiment 31 with the same amount of ethyl oleate (B-9), and replacing the stearic acid (B-5) with the same amount of cyclohexane (B-7), milky lotion 4 (Embodiment 34) was obtained.

### (Embodiment 35, milky lotion 5)

The composition of the milky lotion 5 is shown in Table 4. By preparing, similarly to Embodiment 31: milky lotion 5 (Embodiment 35) was obtained.

**[Table 4]**

| Section | Components (trade name, maker) | % by weight |
|---|---|---|
| a | glycerin (glycerin S, Sakamoto Yakuhin Kogyo Co., Ltd.) | 5.000 |
| | diglycerin (diglycerin 801, Sakamoto Yakuhin Kogyo Co., Ltd.) | 2.000 |
| | 1, 3-butylene glycol (1, 3BG, DAICEL CHEMICAL INDUSTRIES, LTD.) | 7.000 |
| | 1-(2-ethyl hexyl)glycol ether (sencibaSC50, SEIWA KASEI Co., Ltd.) | 0.300 |
| | 1,2-pentandiol (diolPD, Nikko Chemicals Co., Ltd.) | 3.000 |
| | trehalose glucoside (Tornare HAYASHIBARA BIOCHEMICAL LABS. INC.) | 0.500 |
| b | butyl oleate (B-1 0) | 2.000 |
| | 1-bromooctane (B-11) | 2.000 |
| | hexane (B-12) | 2.000 |
| | octane (B-13) | 2.000 |
| | decane (B-14) | 2.500 |
| | natural vitamin E (Riken oil E700, Riken Vitamin co., Ltd.) | 0.010 |
| c | polysaccharide (A-3) | 0.045 |
| | purified water | balance |

### (Embodiment 36: milky lotion 6)

By replacing butyl oleate (B-10) of Section b of Embodiment 35 with the same amount of dodecane (B-15), replacing 1-bromooctane (B-11) with the same amount of oleic acid (B-6), and replacing hexane (B-12) with the same amount of cyclohexane (B-7), milky lotion 6 (Embodiment 36) was obtained.

### (Embodiment 37: milky lotion 7)

The composition of the milky lotion 7 is shown in Table 5.

**[Table 5]**

| Section | Components (trade name, maker) | % by weight |
|---|---|---|
| a | glycerin (glycerin S, Sakamoto Yakuhin Kogyo Co., Ltd.) | 5.00 |
| | xylitol (xylitol, Kanto Chemical Co., Inc.) | 1.00 |
| | 1, 3-butylene glycol (1, 3BG, DAICEL CHEMICAL INDUSTRIES, LTD.) | 8.00 |
| | Esters of p- hydroxybenzoic acid (Mekkins M, UENO FINE CHEMICALS INDUSTRY) | 0.10 |
| b | hexane (B-12) | 4.00 |
| | octane (B-13) | 2.00 |
| | tetradecanol (B-1) | 2.00 |
| | hexadecanol (B-2) | 2.50 |
| | stearic acid (B-5) | 2.00 |
| | natural vitamin E (Riken oil E700, Riken Vitamin Co., Ltd.) | 0.01 |
| c | polysaccharide (A-3) | 0.06 |
| | purified water | balance |
| d | Carboxyvinyl polymer HIVISWAKO105,Wako Pure Chemical Industries, Ltd.) | 0.05 |
| | purified water | 2.45 |
| e | L-Arginine (L-Arginine, AJINOMOTO CO., INC.) | 0.05 |
| | purified water | 0.45 |

The preparing method of the milky lotion 7 is as follows.
1. Polysaccharide (A-3) of Section c was heated to 80°C, and pre-dispersed in water using a disperser (Dispersion liquid 1).
2. Components of Section a were respectively weighed, mixed with the dispersion liquid 1 homogeneously, and dissolved by heating at 80°C (Dispersion liquid 2).
3. Components of Section b were respectively weighed, and dissolved by heating at 80°C (Mixture liquid 1)
4. The mixture liquid 2 was gradually added to the dispersion liquid 2 while agitating the dispersion liquid 2 with a homogenizer (or homomixer) at 8000 rpm (Dispersion liquid 3).
5. HIVISWAKO105 was pre-dispersed in water by using a disperser (Dispersion liquid 4).
6. The dispersion liquid 4 was added to the dispersion liquid 3 and mixed therewith homogeneously (Dispersion liquid 5).
7. Each component of Section e was dispersed homogeneously (Dispersion liquid 6).
8. The dispersion liquid 6 was added to the dispersion liquid 5, after neutralization, they were agitated, and cooled to room temperature, whereby milky lotion 7 (Embodiment 37) was obtained.

### (Comparative example 7: milky lotion 8)

By replacing tetradecanol of Section b of Embodiment 31 with the same amount of decanol (B-18), milky lotion 8 (Comparative example 7) was obtained.

(Comparative example 8: milky lotion 9)
By replacing stearic acid (B-5) of Section b of Embodiment 31 with the same amount of octic acid (B-19), milky lotion 9 (Comparative example 8) was obtained.

### (Embodiment 38: cream 1)

The composition of cream 1 will be shown in Table 6

**[Table 6]**

| Section | Components (trade name, maker) | % by weight |
|---|---|---|
| a | glycerin (glycerin S, Sakamoto Yakuhin Kogyo Co., Ltd.) | 5.00 |
| | (diglycerin (diglycerin 801, Sakamoto Yakuhin Kogyo Co., Ltd.) | 2.00 |
| | 1, 3-butylene glycol (1, 3BG, DAICEL CHEMICAL INDUSTRIES, LTD.) | 7.00 |
| | 1-(2-ethyl hexyl)glycol ether (sencibaSC50, SEIWA KASEI Co., Ltd.) | 0.300 |
| | 1,2-pentandiol (diolPD, Nikko Chemicals Co., Ltd.) | 3.00 |
| | POE methyl glucoside (MagbioburaidoMG-20E, NOF CORPORATION) | 0.50 |
| b | polysaccharide (A-3) | 0.06 |
| | purified water | balance |
| c | octadecanol (B-3) | 3.00 |
| | palmitic acid (B-4)) | 2.00 |
| | ethyl oleate (B-9) | 2.00 |
| | butyl oleate (B-10) | 4.00 |
| | cyclohexane (B-7) | 5.00 |
| | dodecane (B-15) | 9.00 |
| | natural vitamin E (Riken oil E700, Riken Vitamin Co., Ltd.) | 0.05 |
| d | carboxyvinyl polymer(HIVISWAKO104,Wako Pure Chemical Industries, Ltd.) | 0.20 |
| | purified water | 9.80 |
| | carboxyvinyl polymer(HIVISWAKO105,Wako Pure Chemical Industries, Ltd.) | 0.10 |
| | purified water | 4.90 |
| e | L-Arginine (L-Arginine, AJINOMOTO CO., INC.) | 0.30 |
| | purified water | 2.70 |

The preparation method of cream 1 is as follows.
1.Polysaccharide (A-3) of Section b was heated to 80°C, and pre-dispersed in water using a disperser (Dispersion liquid 1).
2. Components of Section a were respectively weighed, mixed with the dispersion liquid 9 homogeneously, and dissolved by heating at 80°C (Dispersion liquid 2).
3. Components of Section c were respectively weighed, and dissolved by heating at 80°C (Mixture liquid 1).
4. The mixture liquid 1 was gradually added to the dispersion liquid 2 while agitating the dispersion liquid 2 with a homogenizer (or homomixer) at 8000 rpm (Dispersion liquid 3).
5. After added, the agitating operation was performed for 10 minutes.
6. HIVISWAKO 104, 105 of Section d were pre-dispersed in water by using a disperser (Dispersion liquid 4).
7. Components of Section e were respectively dispersed homogeneously (Dispersion liquid 5).
8. The dispersion liquid 4 was added to the dispersion liquid 3, and mixed therewith homogeneously (Dispersion liquid 6).
9. The dispersion liquid 5 was added to the dispersion liquid 6, and, after neutralization, they were cooled to room temperature, whereby cream 1 (Embodiment 77) was obtained.

### (Embodiment 39: cream 2)

By replacing carboxyvinyl polymer (HIVISWAKO 104, 105) of Section d of Embodiment 38 with the same amount of hydrated hydroxypropyl methylcellulose (SANGELOSE90L: manufactured by Daido Chemical Corporation), cream 2 (Embodiment 39) was obtained.

### (Embodiment 40: cream 3)

By replacing carboxyvinyl polymer (HIVISWAKO 104, 105) of Section d of Embodiment 38 with the same amount of graft copolymer of starch and sodium acrylate (SANFRESH ST-500D: manufactured by Sanyo Chemical Industries, Ltd), cream 3 (Embodiment 40) was obtained.

### (Embodiment 41: cream 4)

By replacing carboxyvinyl polymer (HIVISWAKO 104, 105) of Section d of Embodiment 77 with the same amount of cellulose crystals (RC-591 S: manufactured by Asahi Kasei Corporation), cream 4 (Embodiment 41) was obtained.

### (Embodiment 42: cream 5)

The composition of cream 5 will be shown in Table 7.

**[Table 7]**

| Section | Components (trade name, maker) | % by weight |
|---|---|---|
| a | purified hydrogenated lecithin (Lecithinol S-10, Nikko Chemicals Co., Ltd.) | 0.20 |
| | glycerin (glycerin S, Sakamoto Yakuhin Kogyo Co., Ltd.) | 5.00 |
| | diglycerin (diglycerin 801, Sakamoto Yakuhin Kogyo Co., Ltd.) | 2.00 |
| | 1, 3-butylene glycol (1, 3BG, DAICEL CHEMICAL INDUSTRIES, LTD.) | 7.00 |
| | 1-(2-ethyl hexyl)glycol ether (sencibaSC50, SEIWA KASEI Co., Ltd.) | 0.30 |
| | 1,2-pentandiol (diolPD, Nikko Chemicals Co., Ltd.) | 3.00 |
| | POE methyl glucoside (MagbioburaidoMG-20E, NOF CORPORATION) | 0.50 |
| b | polysaccharide (A-3) | 0.06 |
| | purified water | balance |
| c | hexadecanol (B-2) | 3.00 |
| | oleic acid (B-6) | 4.00 |
| | ethyl oleate (B-9) | 2.00 |
| | butyl oleate (B-10) | 4.00 |
| | cyclohexane (B-7) | 2.00 |
| | octane (B-13) | 6.00 |
| | dodecane (B-15) | 4.00 |
| | natural vitamin E (Riken oil E700, Riken Vitamin Co., Ltd.) | 0.05 |

The preparation method of cream 7 is as follows:
1.Polysaccharide (A-3) of Section b was heated at 80°C, and pre-dispersed in water using a disperser (Dispersion liquid 1).
2. Components of Section a were respectively weighed, mixed with the dispersion liquid 1 homogeneously, and dissolved by heating at 80°C (Dispersion liquid 2).
3. Components of Section c were respectively weighed, and dissolved by heating at 80°C (Mixture liquid 1)
4. The dispersion liquid 2 was gradually added to the mixture liquid 1 while agitating the mixture liquid 1 with a homogenizer (or homomixer) at 8000 rpm. After added, the agitating operation was further performed for 10 minutes and the cooling operation to room temperature was performed, whereby cream 5 (Embodiment 42) was obtained.

### (Embodiment 43: cream 6)

The composition of cream 6 will be shown in Table 8.

**[Table 8]**

| Section | Components (trade name, maker) | % by weight |
|---|---|---|
| a | polysaccharide (A-3) | 0.064 |
| | purified water | 32.806 |
| b | glycerin (glycerin S, Sakamoto Yakuhin Kogyo Co., Ltd.) | 5.000 |
| | diglycerin (diglycerin 801, Sakamoto Yakuhin Kogyo Co., Ltd.) | 2.000 |
| | 1, 3-butylene glycol (1, 3BG, DAICEL CHEMICAL INDUSTRIES, LTD.) | 7.000 |
| | 1-(2-ethyl hexyl) glycol ether (sencibaSC50, SEIWA KASEI Co., Ltd.) | 0.300 |
| | 1,2-pentandiol (diolPD, Nikko Chemicals Co., Ltd.) | 3.000 |
| | Purified water | balance |
| c | hexadecanol (B-2) | 6.000 |
| | oleic acid (B-6) | 2.000 |
| | ethyl oleate (B-9) | 4.000 |
| | butyl oleate (B-10) | 1.000 |
| | Cyclohexane (B-7) | 4.000 |
| | octane (B-13) | 0.500 |
| | dodecane (B-15) | 2.000 |
| | Stearic acid (B-5) | 8.000 |
| | natural vitamin E (Riken oil E700, Riken Vitamin co., Ltd.) | 0.030 |
| d | Carboxyvinyl polymer (HIVISWAKO104,Wako Pure Chemical Industries, Ltd.) | 0.200 |
| | 1, 3-butylene glycol (1, 3BG, DAICEL CHEMICAL INDUSTRIES, LTD.) | 2.000 |
| | purified water | 7.800 |
| e | alkyl copolymer of acrylate and methacrylate (PemulensTR-2, B.F.Goodrich Company) | 0.080 |
| | 1, 3-butylene glycol (1, 3BG, DAICEL CHEMICAL INDUSTRIES, LTD.) | 0.800 |
| | purified water | 3.120 |
| f | dipotassium glycyrrhizinate (GLYTIONON K2, TOKIWA T o T TOKIWA Phytochemical Co., Ltd.) | 0.100 |
| | purified water | 3.120 |
| g | L-Arginine (I-Arginine, AJINOMONOT CO., INC.) | 0.600 |
| | purified water | 5.400 |
| h | L-Ascorbic acid 2-glucoside (AS-G, HAYASHIBARA BIOCHEMICAL LABS., INC.) | 2.000 |
| | triethanolamine | 1.000 |
| | purified water | 10.000 |

The preparing method of cream 6 is as follow:
1. Polysaccharide (A-3) of Section a was heated at 80°C, and pre-dispersed in water using a disperser (Dispersion liquid 1).
2. Components of Section b were respectively weighed, and dissolved by heating at 70°C (Mixture liquid 1).
3. Components of Section c were respectively weighed, and dissolved by heating at 70°C(Mixture liquid 2)
4. Components of Section d and Section e were respectively weighed, homogeneously dissolved, and homogeneously mixed (Mixture liquid 3)
5. The mixture liquid 1 was gradually added to the dispersion liquid 1 at 70°C while agitating the mixture liquid 1 with a homogenizer (or homomixer) at 8000 rpm, and the mixture liquid 2 was gradually added at 70°C.
6. After added, the heating and agitating operation was further performed for 10 minutes (Dispersion liquid 2).
7. The mixture liquid 2 was added to the dispersion liquid 2, and mixed homogeneously (Dispersion liquid 3).
8. Components of Section f, Section g, and Section h were respectively weighed, and homogeneously dissolved. Then, they were added to the dispersion liquid 3, agitated and cooled to room temperature, whereby cream 6 (Embodiment 84) was obtained.

### (Comparative example 9: cream 7)

By replacing the octadecanol (B-3) of Section c of Embodiment 38 with the same amount of hexanol (B-16), cream 7 (Comparative example 9) was obtained.

### (Embodiment 44: sunscreen 1)

The composition of sunscreen 1 is shown in Table 9.

**[Table 9]**

| Section | Components (trade name, maker) | % by weight |
|---|---|---|
| a | titanium dioxide (MT-01*, TAYCA Co.,Ltd.) | 10.000 |
| | silicone (Shin-etsu silicon KF96-100, Shin-Etsu Chemical Co., Ltd.) | 15.000 |
| b | hydrogenated lecithin (LecithinoISH50, Nikko Chemicals Co., Ltd.) | 3.000 |
| | 1, 3-butylene glycol (1, 3BG, DAICEL CHEMICAL INDUSTRIES, LTD.) | 15.000 |
| | glycerin (glycerin S, Sakamoto Yakuhin Kogyo Co., Ltd.) | 15.000 |
| | phenoxyethanol (PHENOXYETHANOL S, Lion corporation) | 0.300 |
| c | hexadecanol (B-2) | 1.500 |
| | oleic acid (B-6) | 7.000 |
| | ethyl oleate (B-9) | 2.000 |
| | dodecane (B-15) | 5.00 |
| d | polysaccharide (A-3) | 0.045 |
| | purified water | balance |

| | | |
|---|---|---|
| * MT-01 surface treatment with aluminum hydroxide stearic acid | | |

The preparing method of sunscreen 1 is as follow:
1.Polysaccharide (A-3) of Section d was heated at 80°C, and pre-dispersed in water using a disperser (Dispersion liquid 1).
2. Components of Section a were respectively weighed, mixed homogeneously, and dissolved by heating at 80°C (Dispersion liquid 2).
3. Components of Section b were respectively weighed, mixed homogeneously, and dissolved by heating at 80°C (Dispersion liquid 3)
4. The dispersion liquid 3 was gradually added to the dispersion liquid 2 while heating and agitating the same (Dispersion liquid 4).
5. Components of Section c were respectively weighed, mixed homogeneously, and dissolved by heating at 80°C (Dispersion liquid 5)
6. The dispersion liquid 5 was gradually added to the dispersion liquid 1 while agitating the dispersion liquid 1 with a homogenizer (or homomixer) at 8000 rpm, and then, the dispersion liquid 4 was gradually added.
5. After added, a resultant mixture was further agitated for 10 minutes, and then cooled to room temperature, thereby obtaining sunscreen 1 (Embodiment 44).

### (Embodiment 45: Sunscreen 2)

The composition of sunscreen 2 is shown in Table 10.

**[Table 10]**

| Section | Components (trade name, maker) | % by weight |
|---|---|---|
| a | glycerin (glycerin S, Sakamoto Yakuhin Kogyo Co., Ltd.) | 5.000 |
| | 1, 3-butylene glycol (1, 3BG, DAICEL CHEMICAL INDUSTRIES, LTD.) | 7.000 |
| | 1-(2-ethyl hexyl)glycol ether (sencibaSC50, SEIWA KASEI Co., Ltd.) | 0.300 |
| | 1,2-pentandiol (diolPD, Nikko Chemicals Co., Ltd.) | 3.000 |
| | titanium dioxide (SMT-100WR, TAYCA Co.,Ltd.) | 7.000 |
| b | hexadecanol (B-2) | 4.000 |
| | oleic acid (B-6) | 2.000 |
| | ethyl oleate (B-9) | 2.000 |
| | dodecane (B-15) | 1.500 |
| | tetradecanol (B-1) | 2.000 |
| | octadecanol (B-3) | 2.500 |
| | cyclohexane (B-7) | 2.000 |
| | natural vitamin E (Riken oil E700, Riken Vitamin co., Ltd.) | 0.010 |
| c | polysaccharide (A-3) | 0.045 |
| | purified water | balance |

The preparation method of sunscreen 2 is as follows.
1.Polysaccharide (A-3) of Section c was heated to 80°C, and pre-dispersed in water using a disperser (Dispersion liquid 1).
2. Components of Section a were respectively weighed, mixed homogeneously with the dispersion liquid 1, and dissolved by heating at 80°C (Dispersion liquid 2).
3. Components of Section b were respectively weighed, and dissolved by heating at 80°C (Mixture liquid 1)
4. The mixture liquid 1 was gradually added to the dispersion liquid 2 while agitating the same with a homogenizer (or homomixer) at 8000 rpm. After added, the agitation operation was further performed, and the cooling operation was performed to room temperature, thereby obtaining sunscreen 2 (Embodiment 45).

### (Comparative example 10: sunscreen 3)

By replacing the oleic acid (B-6) of Section b of Embodiment 45 with the same amount of cyclohexanol (B-22), sunscreen 3 (Comparative example 10) was obtained.

### (Stability test of cosmetic)

The cosmetics of Embodiments 31 through 45 and Comparative examples 7 through 10 were prepared, and then, 100 ml of each cosmetic was sampled in a graduated cylinder with ground-in stopper of 100 ml, the cylinders were sealed and allowed to stand in a thermostatic chamber at 45°C, and after 12 weeks, the separation in the cosmetic within the graduated cylinder with ground-in stopper of 100 ml was observed visually. The test results were shown in Table 14 according to the following evaluation criteria. All embodiments show good results.

### (Evaluation criteria of stability)

○: separation and precipitation are not observed visually.
×: separation and precipitation are observed visually

### [Sensory test of Cosmetic (Evaluation of feeling upon using)]

The cosmetics (Embodiments 31 through 45 and Comparative examples 7 through 10) just after preparation, and the cosmetics (Embodiments 31 through 45 and Comparative examples 7 through 10) allowed to stand in the thermostatic chamber at 45°C continuously for 12 weeks were respectively subdivided in ten sets of vessels, each having an identical external appearance, such that they cannot be distinguished from each other. Two panelers in every ages from teens to fifties, ten panelers in total, were selected, a proper amount of each of the cosmetics was taken with fingers, spread on backs of both hands of each paneler, and sensory evaluation on "smoothness" thereof was carried out. The evaluation criteria of "smoothness" were determined as follows. The results were shown in Table 11. All embodiments show good results.

### (Evaluation criteria of "smoothness")

○: evaluated by eight or more out of ten panelers to have smooth feel
X: evaluated by seven or less out of ten panelers to have smooth feel

**[Table 11]**

| Example | Cosmetic | Separation and precipitation | Smoothness | |
|---|---|---|---|---|
| | | | Just after preparation | After 12 weeks |
| Embodiment 31 | milky lotion 1 | ○ | ○ | ○ |
| Embodiment 32 | milky lotion 2 | ○ | ○ | ○ |
| Embodiment 33 | milky lotion 3 | ○ | ○ | ○ |
| Embodiment 34 | milky lotion 4 | ○ | ○ | ○ |
| Embodiment 35 | milky lotion 5 | ○ | ○ | ○ |
| Embodiment 36 | milky lotion 6 | ○ | ○ | ○ |
| Embodiment 37 | milky lotion 7 | ○ | ○ | ○ |
| Embodiment 38 | cream 1 | ○ | ○ | ○ |
| Embodiment 39 | cream 2 | ○ | ○ | ○ |
| Embodiment 40 | cream 3 | ○ | ○ | ○ |
| Embodiment 41 | cream 4 | ○ | ○ | ○ |
| Embodiment 42 | cream 5 | ○ | ○ | ○ |
| Embodiment 43 | cream 6 | ○ | ○ | ○ |
| Embodiment 44 | sunscreen 1 | ○ | ○ | ○ |
| Embodiment 45 | sunscreen 2 | ○ | ○ | ○ |
| Comparative example 7 | milky lotion 8 | X | ○ | X |
| Comparative example 8 | milky lotion 9 | X | ○ | X |
| Comparative example 9 | cream 7 | X | ○ | X |
| Comparative example 10 | sunscreen 3 | X | ○ | X |

## Claims

1. A cosmetic containing an emulsification-dispersing agent composed of a polysaccharide having a particulate structure as a main component, and a component to be emulsification-dispersed, wherein said component to be emulsification-dispersed is composed of two or more kinds of components to be emulsification-dispersed, each having a dielectric constant ranging from 1 (F/m) to 5 (F/m), and an inorganicity/organicity ratio ranging from 0 to 0.5.

2. A cosmetic as claimed in claim 1, wherein said emulsification-dispersing agent composed of said polysaccharide having said particulate structure as said main component adheres to a periphery of said component to be emulsification-dispersed into a layer, thereby defining an intermediate layer.

3. A cosmetic as claimed in claim 1, wherein said polysaccharide with said particulate structure has an average particle diameter ranging from 8 nm to 500 nm.

4. A cosmetic as claimed in claim 1, wherein said polysaccharide with said particulate structure is a polysaccharide composed of a monoparticle.

5. A cosmetic as claimed in claim 1, wherein said polysaccharide with said particulate structure contains at least one of fucose, glucose, glucuronic acid and rhamnose as a constituent monosaccharide, and further contains fucose and/or rhamnose in a side chain thereof.

6. A cosmetic as claimed in claim 5, wherein said polysaccharide with said particulate structure includes at least a polysaccharide represented by the following formula (1).

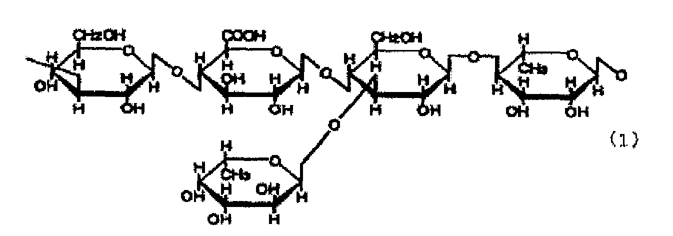

7. A cosmetic as claimed in claim 1, wherein said emulsification-dispersing agent containing said polysaccharide with said particulate structure as said main component, and a component to be emulsification-dispersed exist in the weight ratio of from 1 : 50 to 1: 1000.

8. A cosmetic as claimed in claim 1, wherein the content of said polysaccharide with said particulate structure ranges from 0.001% by weight to 1 % by weight relative to the total amount of the cosmetic.

9. A cosmetic as claimed in claim 1, wherein said component to be emulsification-dispersed is composed of two or more kinds of a lake pigment, organic pigment, color pigment, white pigment, inorganic pigment such as an extender pigment, nacreous pigment, metallic luster pigment, silica frit pigment, metal coated inorganic pigment, resin pigment, high polymer powders, functional pigment.

10. A cosmetic as claimed in claim 1, wherein said cosmetic does not contain any one of a cationic surfactant, an anionic surfactant and a nonionic surfactant composed of alkylene(carbon atoms: 2 to 4) oxide adduct.

11. A cosmetic as claimed in claim 1, wherein said polysaccharide is granulated to have randomly scattering particle diameters.

12. A cosmetic as claimed in claim 1, wherein said cosmetic contains urea in an amount ranging from 0.1 % by weight to 10 % by weight relative to the total amount of the cosmetic.
